(19) Europäisches Patentamt
European Patent Office
Office européen des brevets

(11) Veröffentlichungsnummer : **0 097 270**
**B1**

(12) **EUROPÄISCHE PATENTSCHRIFT**

(45) Veröffentlichungstag der Patentschrift : **28.08.85**

(21) Anmeldenummer : **83105481.2**

(22) Anmeldetag : **03.06.83**

(51) Int. Cl.[4] : **C 07 F 9/12**, C 07 F 9/18, C 07 F 9/24, C 07 C149/36, C 07 C147/10, A 01 N 57/14, A 01 N 57/30

---

(54) **Organophosphat-Derivate.**

---

(30) Priorität : **18.06.82 JP 103908/82**

(43) Veröffentlichungstag der Anmeldung : **04.01.84 Patentblatt 84/01**

(45) Bekanntmachung des Hinweises auf die Patenterteilung : **28.08.85 Patentblatt 85/35**

(84) Benannte Vertragsstaaten : **AT BE CH DE FR GB IT LI NL**

(56) Entgegenhaltungen :
**DE-A- 1 934 001**
**DE-A- 2 625 764**
**FR-A- 1 173 264**
**FR-A- 2 385 726**
**US-A- 3 755 511**

(73) Patentinhaber : **NIHON TOKUSHU NOYAKU SEIZO K.K.**
**No.4, 2-Chome, Nihonbashi Honcho**
**Chuo-ku Tokyo 103 (JP)**

(72) Erfinder : **Saito, Junichi**
**3-7-12, Osawa**
**Mitaka-shi Tokyo (JP)**
Erfinder : **Kudamatsu, Akio**
**179, Kariyado Nakahara-ku**
**Kawasaki-shi Kanagawa-ken (JP)**
Erfinder : **Kume, Toyohiko**
**6-7-8, Asahigaoka**
**Hino-shi Tokyo (JP)**
Erfinder : **Tsuboi, Shinichi**
**3-26-1, Hirayama**
**Hino-shi Tokyo (JP)**

(74) Vertreter : **Schumacher, Günter, Dr. et al**
**c/o Bayer AG Konzernverwaltung RP Patentabteilung**
**D-5090 Leverkusen 1 Bayerwerk (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 097 270 B1

Jouve, 18, rue St-Denis, 75001 Paris, France

**Beschreibung**

Die vorliegende Erfindung betrifft Organophosphat-Derivate, Verfahren zu ihrer Herstellung und ein insektizides, akarizides (mitizides) oder nematozides Mittel.

Die vorliegende Erfindung betrifft Organophosphat-Derivate, die durch die nachstehende Formel (I) dargestellt sind

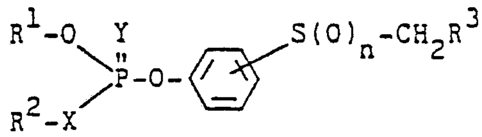

(I)

in der

X für O, S oder NH steht,

Y für O oder S steht,

$R^1$ eine niedere Alkyl-Gruppe bezeichnet,

$R^2$ eine niedere Alkyl-Gruppe oder eine niedere Alkoxy-Niederalkyl-Gruppe bezeichnet,

$R^3$ eine fluoro-substituierte niedere Alkyl-Gruppe bezeichnet und

n für 0 oder 2 steht.

Die Verbindungen der allgemeinen Formel (I) können beispielsweise mittels der folgenden Verfahren hergestellt werden, auf die sich die vorliegende Erfindung ebenfalls bezieht.

Verfahren i)

Ein Verfahren zur Herstellung der Organophosphat-Derivate der allgemeinen Formel (I) ist dadurch gekennzeichnet, daß ein Phosphorsäureester-halogenid der allgemeinen Formel (II)

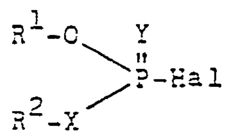

(II)

in der

X, Y, $R^1$ und $R^2$ die im Vorstehenden angegebenen Bedeutungen haben und

Hal ein Halogen-Atom bezeichnet,

mit einem Phenol der allgemeinen Formel (III)

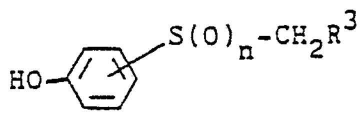

(III)

in der

$R^3$ und n die im Vorstehenden angegebenen Bedeutungen haben,

zur Reaktion gebracht wird.

Verfahren ii) (X = S)

Ein Verfahren zur Herstellung eines Organophosphat-Derivats der allgemeinen Formel (I')

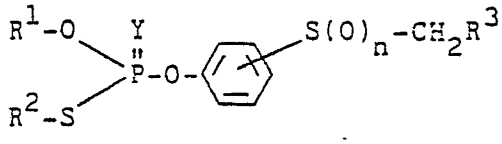

(I')

in der

Y, $R^1$, $R^2$, $R^3$ und n die im Vorstehenden angegebenen Bedeutungen haben,

ist dadurch gekennzeichnet, daß ein Phosphorsäure-Salz der allgemeinen Formel (IV)

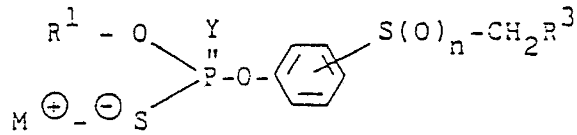

(IV)

in der
Y, $R^1$, $R^3$ und n die im Vorstehenden angegebenen Bedeutungen haben und
M für ein Alkalimetall oder eine Ammonium-Gruppe steht,
mit einem Halogenid der allgemeinen Formel (V)

$R^2$—Hal (V)

in der
$R^2$ und Hal die im Vorstehenden angegebenen Bedeutungen haben,
zur Reaktion gebracht wird.

Die Organophosphat-Derivate der allgemeinen Formel (I) gemäß der vorliegenden Erfindung können auch mittels des folgenden Verfahrens iii) hergestellt werden, und solche Derivate der allgemeinen Formel (I), in denen n 2 ist, können auch mittels des folgenden Verfahrens iv) hergestellt werden.

Verfahren iii)

Ein Verfahren zur Herstellung der Organophosphat-Derivate der allgemeinen Formel (I) ist dadurch gekennzeichnet, daß ein Phosphorsäureester-halogenid der allgemeinen Formel (VI)

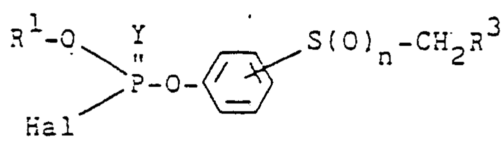

(VI)

in der
Y, $R^1$, $R^3$, n und Hal die im Vorstehenden angegebenen Bedeutungen haben,
mit einer Verbindung der allgemeinen Formel (VII)

$R^2$—X—H (VII)

in der
X und $R^2$ im Vorstehenden angegebenen Bedeutungen haben,
zur Reaktion gebracht wird.

Verfahren iv) (n = 2)

Ein Verfahren zur Herstellung von Organophosphat-Derivaten der allgemeinen Formel (I-b)

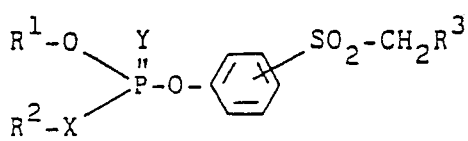

(I-b)

in der
X, Y, $R^1$, $R^2$ und $R^3$ die im Vorstehenden angegebenen Bedeutungen haben,
ist dadurch gekennzeichnet, daß ein Organophosphat-Derivat der allgemeinen Formel (I-a)

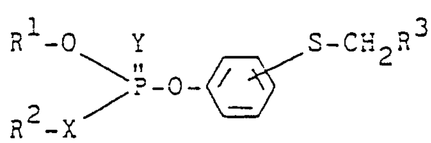

(I-a)

in der

X, Y, $R^1$, $R^2$ und $R^3$ die im Vorstehenden angegebenen Bedeutungen haben, mit einem Peroxid zur Reaktion gebracht wird.

Die vorliegende Erfindung betrifft außerdem ein insektizides, akarizides oder nematozides Mittel, das das Organophosphat-Derivat der allgemeinen Formel (I) als Wirkstoff enthält.

Die vorliegende Erfindung betrifft weiterhin das Phenol-Derivat der allgemeinen Formel (III), das ein Zwischenprodukt für die Herstellung der Verbindungen der allgemeinen Formel (I) ist.

Das Phenol-Derivat der allgemeinen Formel (III) kann beispielsweise mittels der folgenden Verfahren v) und vi) hergestellt werden, auf die sich die vorliegende Erfindung ebenfalls bezieht.

Verfahren v) (n = 0)

Ein Verfahren zur Herstellung eines Phenol-Derivats der allgemeinen Formel (III-a)

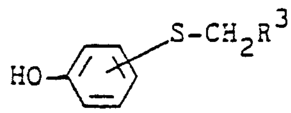

(III-a)

in der $R^3$ die im Vorstehenden angegebene Bedeutung hat, ist dadurch gekennzeichnet, daß ein Sulfonat der allgemeinen Formel

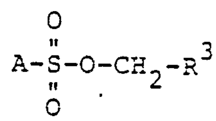

(VIII)

in der
$R^3$ die im Vorstehenden angegebene Bedeutung hat und
A eine Alkyl- oder Aryl-Gruppe bezeichnet,
mit einem Hydroxyphenylthiol der Formel (IX)

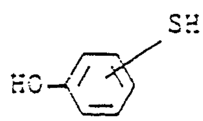

(IX)

zur Reaktion gebracht wird.

Verfahren vi) (n = 2)

Ein Verfahren zur Herstellung eines Phenol-Derivats der allgemeinen Formel (III-b)

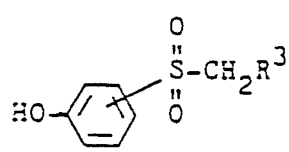

(III-b)

in der $R^3$ die im Vorstehenden angegebene Bedeutung hat, ist dadurch gekennzeichnet, daß ein Phenol-Derivat der allgemeinen Formel (III-a)

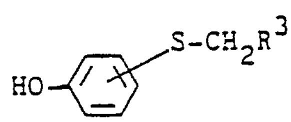

(III-a)

in der
$R^3$ die im Vorstehenden angegebene Bedeutung hat,
mit einem Peroxid zur Reaktion gebracht wird.

Die US-PS 3 755 511, eine vor der Einreichtung der vorliegenden Anmeldung bekannte Druckschrift, offenbart, daß Verbindungen der allgemeinen Formel (X) herbizide, fungizide und insektizide Aktivität besitzen.

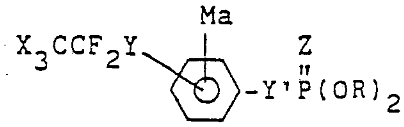

(X)

In der Formel (X) bezeichnen

X jeweils unabhängig voneinander Wasserstoff, Brom, Chlor oder Fluor, jedoch mit der Maßgabe, daß wenigstens ein X immer Brom, Chlor oder Fluor ist,

Y, Y′ und Z jeweils unabhängig voneinander O oder S,

M jeweils unabhängig voneinander Brom, Chlor, Fluor, Iod, Nitro oder eine niedere Alkyl-Gruppe mit 1 bis 4 Kohlenstoff-Atomen,

a 0, 1, 2 oder 3 und

R jeweils unabhängig voneinander eine niedere Alkyl-Gruppe mit 1 bis 4 Kohlenstoff-Atomen.

Die US-PS 4 139 615 (entsprechend der JP-OS 151151/1977) gibt an, daß Verbindungen der allgemeinen Formel (XI)

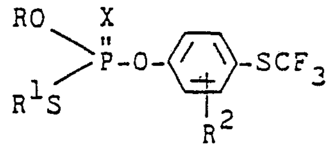

(XI)

in der

R und $R^1$ jeweils eine Alkyl-Gruppe bezeichnen,

$R^2$ ein Wasserstoff-Atom oder eine Alkyl-Gruppe bezeichnet und

X für ein Sauerstoff- oder Schwefel-Atom steht, insektizide, akarizide und nematozide Aktivität besitzen.

Seitens der Anmelderin wurden ausgedehnte Untersuchungen über Organophosphate durchgeführt, die typische insektizide Verbindungen sind. Bei diesen Untersuchungen wurde gefunden, daß die Organophosphat-Derivate der allgemeinen Formel (I), in unerwarteter Weise hervorragende biologische Aktivitäten zeigen.

Die von der Anmelderin durchgeführten Untersuchungen haben ergeben, daß die aktiven Verbindungen der Formel (I) der vorliegenden Erfindung eine ausgezeichnete Bekämpfungswirkung gegen Schadinsekten, Milben und Nematoden in der Landwirtschaft, Forstwirtschaft und im Gartenbau zeigen und daß ihre Wirksamkeit als Schädlingsbekämpfungsmittel in der Praxis bei weitem die Wirkung der Verbindungen übertrifft, die in den beiden im Vorstehenden zitierten, bekannten Veröffentlichungen beschrieben sind und strukturell den Verbindungen der vorliegenden Erfindung ähnlich sind.

Das hervorstechende Merkmal der chemischen Struktur der Verbindungen der Formel (I) der vorliegenden Erfindung besteht darin, daß die Phenyl-Gruppe des O-Phenylesters in der Phosphat-Struktur in einer beliebigen Stellung, vorzugsweise in der 2- oder der 4-Stellung und besonders bevorzugt in der 4-Stellung, durch die $-CH_2R_3$-Gruppe über ein Schwefel-Atom oder ein Sulfonyl-Gruppe substituiert ist. Es wurde gefunden, daß die Verbindungen gemäß der vorliegenden Erfindung eine überraschende, bisher nicht beobachtete Bekämpfungswirkung gegenüber den im Vorstehenden erwähnten Organismus auszuüben vermögen.

Die Phenol-Derivate der allgemeinen Formel (III) sind industriell als Zwischenprodukte für die Herstellung der aktiven Verbindungen der allgemeinen Formel (I) von Wert.

Die aktiven Verbindungen der vorliegenden Erfindung zeigen eine genaue Bekämpfungswirkung gegen schädliche Insekten, Milben und Nematoden, ohne daß sie in irgendeiner Weise phytotoxisch gegen Kulturpflanzen wirken. Weiterhin können die Verbindungen gemäß der vorliegenden Erfindung zur Kontrolle und Bekämpfung einer großen Mannigfaltigkeit von Schädlingen eingesetzt werden, darunter saugende und beißende Schadinsekten, andere Pflanzenparasiten, Schädlinge von Lagergetreide und Schädlinge, die auf dem Gebiet der Hygiene von Bedeutung sind.

Spezielle Beispiele für diese Schädlinge sind :

Insekten der Ordnung Coleoptera, wie etwa
- Callosobruchus chinensis,
- Sitophilus zeamais,
- Tribolium castaneum,
- Epilachna vigintioctomaculata
- Agriotes fuscicollis,
- Anomala rufocuprea
- Leptinotarsa decemlineata,
- Diabrotica spp.,
- Monochamus alternatus und
- Lyctus brunneus ;

Insekten der Ordnung Lepidoptera, wie etwa
- Lymantria dispar,
- Malacosoma neustria,
- Pieris rapae,
- Spodoptera litura,
- Mamestra brassicae
- Chilo suppressalis,
- Pyrausia nubilalis,
- Ephestia cautella,
- Adoxophyes orana,
- Carpocapsa pomonella,
- Galleria mellonella und
- Phyllocnistis citrella ;

Insekten der Ordnung Hemiptera, wie etwa
- Nephotettix cincticeps,
- Nilaparvata lugens,
- Pseudococcus comstocki,
- Unaspis yanonensis,
- Myzus persicae,
- Aphis pomi,
- Rhopalosiphum pseudobrassicae,
- Stephanitis nashi,
- Nazara spp.,
- Cimex lectularius,
- Trialeurodes vaporariorum und
- Psylla spp. ;

Insekten der Ordnung Orthoptera, wie etwa
- Blatella germanica,
- Periplaneta americana,
- Gryllotalpa africana und
- Locusta migratoria migratoriodes ;

Insekten der Ordnung Isoptera, wie etwa
- Deucotermes speratus und
- Coptotermes formosanus ; sowie

Insekten der Ordnung Diptera, wie etwa
- Musca domestica,
- Aedes aegypti,
- Hylemia platura,
- Culex pipiens,
- Anopheles sinensis,
- Culex tritaeniorhynchus ;

Milben, wie etwa
- Tetranychus telarius,
- Panonychus citri,
- Aculus pelekassi und
- Torronomus spp. und

Nematoden, wie etwa
- Meloidogyne incognita,
- Bursaphelenchus lignicolus Mamiya et Kiyohara,
- Aphelenchoides besseyi,
- Heterodera glycines und
- Pratyleochus spp.

Auf dem Gebiet der Veterinärmedizin können die neuen Verbindungen gemäß der vorliegenden Erfindung gegenüber verschiedenen Tierparasiten (inneren und äußeren Parasiten) wie Zecken, Insekten und Würmern wirksam verwendet werden.

Als spezielle Beispiele für solche Zecken seien
- Oranithodoros spp.,
- Ixodes spp. und
- Boophilus spp.

Als spezielle Beispiele für solche Insekten
Gastrophilus spp.,
Stomoxys spp.,
Trichodectes spp.,
Rhodnius spp. und
Ctenocephalidex canis
erwähnt.

Die Verbindungen der Formel (I) gemäß der vorliegenden Erfindung können beispielsweise mittels des folgenden Verfahrens hergestellt werden :

Verfahren i)

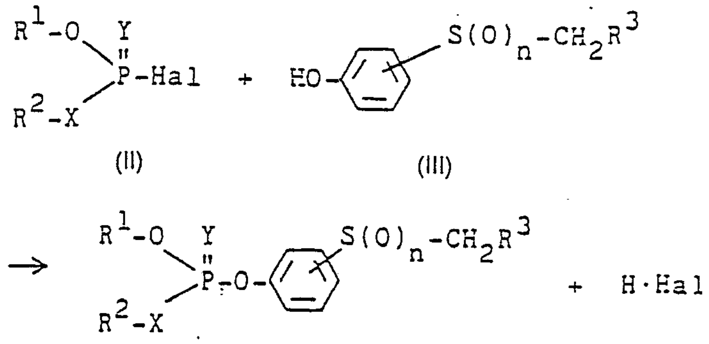

(II) (III)

(I)

In dem vorstehenden Reaktionsschema bezeichnen
X für O, S oder NH,
Y für O oder S,
$R^1$ eine niedere Alkyl-Gruppe, beispielsweise eine Alkyl-Gruppe mit 1 bis 4 Kohlenstoff-Atomen wie Methyl, Ethyl, Propyl, Isopropyl und n-, iso-, sec- oder tert-Butyl,
$R^2$ eine niedere Alkyl-Gruppe oder eine niedere Alkoxy-Niederalkyl-Gruppe, beispielsweise die gleiche niedere Alkyl-Gruppe, wie sie im Vorstehenden beispielhaft genannt ist, oder die gleiche niedere Alkyl-Gruppe, wie sie im Vorstehenden beispielhaft genannt ist, die durch eine niedere Alkoxy-Gruppe mit der gleichen niederen Alkyl-Gruppe, wie sie im Vorstehenden beispielhaft genannt ist, substituiert ist,
$R^3$ eine fluoro-substituierte niedere Alkyl-Gruppe, beispielsweise die gleiche niedere Alkyl-Gruppe, wie sie im Vorstehenden beispielhaft genannt ist, die durch ein Fluor-Atom substituiert ist, und
n 0 oder 2 ;
Hal steht für ein Halogen-Atom wie Fluor, Chlor, Brom oder Iod, vorzugsweise für Chlor, Brom und Iod.

Zu speziellen Beispielen für das Phosphorsäureesterhalogenid der allgemeinen Formel (II), das als Ausgangsmaterial bei dem Verfahren zur Herstellung der Verbindungen, das durch das vorstehende Reaktionsschema dargestellt ist, eingesetzt wird, zählen

O-Ethyl-S-propyl-thiophosphat-chlorid,
O-Ethyl-S-propyl-dithiophosphat-chlorid,
O,O-Diethyl-thiophosphat-chlorid,
O,O-Dimethyl-thiophosphat-chlorid,
O-Ethyl-S-sec-butyl-thiophosphat-chlorid,
O-Ethyl-S-ethoxyethyl-thiophosphat-chlorid,
O-Ethyl-N-isopropylamido-thiophosphat-chlorid sowie
an Stelle der vorstehenden Phosphorsäureester-chloride die entsprechenden Bromide.

Zu Beispielen für das Phenol der allgemeinen Formel (III), die in gleicher Weise Ausgangsmaterialien sind, zählen

4-(2,2,2-Trifluoroethylthio) phenol,
2-(2,2,2-Trifluoroethylthio) phenol,
4-(2,2,2-Trifluoroethylsulfonyl) phenol,
4-(2,2,3,3-Tetrafluoropropylthio) phenol,
2-(2,2,3,3-Tetrafluoropropylthio) phenol,
4-(2,2,3,3,3-Pentafluoropropylthio) phenol und
4-(2,2,3,3-Tetrafluoropropylsulfonyl) phenol.

Das vorerwähnte Herstellungsverfahren wird anhand eines typischen Beispiels im einzelnen erläutert.

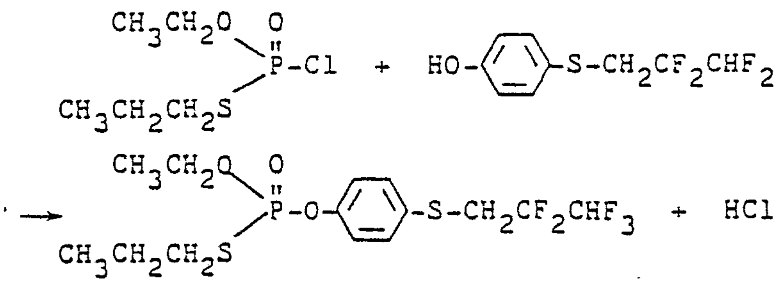

Das Verfahren zur Herstellung der aktiven Verbindungen gemäß der vorliegenden Erfindung kann zweckmäßigerweise unter Verwendung eines Lösungsmittels oder Verdünnungsmittels durchgeführt werden. Zu diesem Zweck können alle inerten Lösungsmittel oder Verdünnungsmittel verwendet werden.

Beispiele für solche Lösungsmittel oder Verdünungsmittel sind Wasser ; aliphatische, alicyclische und aromatische Kohlenwasserstoffe (die gegebenenfalls chloriert sein können) wie Hexan, Cyclohexan, Petrolether, Ligroin, Benzol, Toluol, Xylol, Methylenchlorid, Chloroform, Kohlenstofftetrachlorid, Ethylenchlorid, Trichloroethylen und Chlorbenzol ; Ether wie Diethylether, Methylethylether, Di-isopropylether, Dibutylether, Propylenoxid, Dioxan und Tetrahydrofuran ; Ketone wie Aceton, Methylethylketon, Methylisopropylketon und Methylisobutylketon ; Nitrile wie Acetonitril, Propionitril und Acrylnitril ; Alkohole wie Methanol, Ethanol, Isopropanol, Butanol und Ethylenglycol ; Ester wie Ethylacetat und Amylacetat ; Säureamide wie Dimethylformamid und Dimethylacetamid ; Sulfone und Sulfoxide wie Dimethylsulfoxid und Sulfolan ; sowie Basen wie Pyridin.

Die Reaktion kann in Gegenwart eines säurebindenden Mittels durchgeführt werden. Beispiele für solche säurebindenden Mittel sind die Hydroxide, Carbonate, Hydrogencarbonate und Alkoholate von Alkalimetallen sowie tertiäre Amine wie Triethylamin, Diethylanilin und Pyridin, die allgemein verwendet werden.

Das Verfahren gemäß der vorliegenden Erfindung kann bei einer Temperatur innerhalb eines weiten Bereichs durchgeführt werden, im allgemeinen bei einer Temperatur zwischen etwa – 20 °C und dem Siedepunkt der Mischung, zweckmäßigerweise zwischen etwa 0° C und etwa 100° C. Zweckmäßigerweise wird die Reaktion unter atmosphärischem Druck durchgeführt, jedoch ist es auch möglich, unter erhöhtem oder vermindertem Druck zu arbeiten.

Verfahren ii) (X = S)

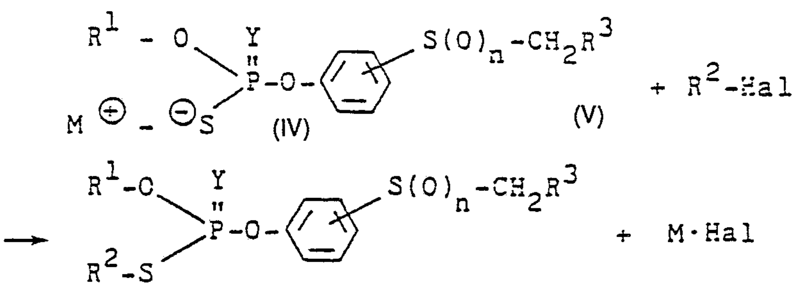

(IV) (V)

(I')

In dem vorstehenden Reaktionsschema sind Beispiele für Y, $R^1$, $R^2$, $R^3$, n und Hal die gleichen, die im Vorstehenden angegeben sind.

Zu Beispielen für M zählen Alkalimetall-Atome wie Lithium, Kalium und Natrium und eine Ammonium-Gruppe.

Zu speziellen Beispielen für das Phosphorsäure-Salz der allgemeinen Formel (IV), die als Ausgangsmaterial bei dem Verfahren, das durch das vorstehende Reaktionsschema dargestellt ist, eingesetzt werden, zählen

Kalium-O-ethyl-O-[4-(2,2,2-trifluoroethylthio)-phenyl] thiophosphat,
Kalium-O-ethyl-O-[2-(2,2,2-trifluoroethylthio)-phenyl] thiophosphat,
Kalium-O-ethyl-O-[4-(2,2,3,3-tetrafluoropropylthio) phenyl] thiophosphat,
Kalium-O-ethyl-O-[4-(2,2,3,3,3-pentafluoropropylthio) phenyl] thiophosphat,
Kalium-O-ethyl-O-[2-(2,2,3,3-tetrafluoropropylthio) phenyl] thiophosphat,
Kalium-O-ethyl-O-[4-(2,2,2-trifluoroethylsulfonyl)-phenyl] thiophosphat und
Kalium-O-ethyl-O-[4-(2,2,3,3-tetrafluoropropylsulfonyl) phenyl] thiophosphat.

Die entsprechenden Alkalimetall-Salze, speziell die Lithium- oder Natrium-Salze, und die Ammonium-Salze sind neben den Kalium-Salzen ebenfalls zu nennen.

Zu Beispielen für das Halogenid der allgemeinen Formel (V), die in gleicher Weise Ausgangsmaterialien sind, zählen Propylbromid, sec-Butylbromid und 2-Ethoxyethylbromid. Die entsprechenden Chloride sind ebenfalls zu nennen.

Das vorerwähnte Herstellungsverfahren wird anhand eines typischen Beispiels im einzelnen erläutert.

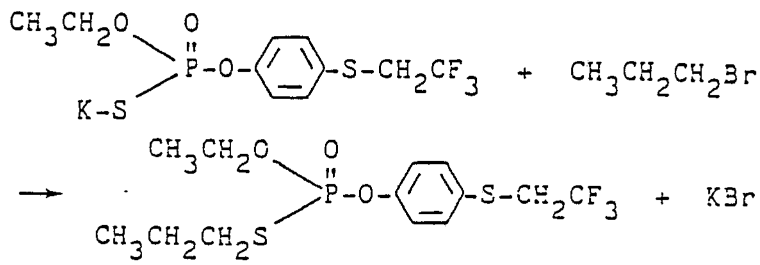

Das vorstehende Verfahren kann bei einer Temperatur innerhalb eines weiten Bereichs unter Verwendung der gleichen inerten Lösungsmittel oder Verdünnungsmittel, wie sie im Vorstehenden beispielhaft angegeben sind, durchgeführt werden. Die Reaktion kann allgemein bei einer Temperatur zwischen etwa – 20 °C und dem Siedepunkt der Mischung, zweckmäßigerweise zwischen etwa 0 °C und etwa 100 °C, durchgeführt werden. Zweckmäßigerweise wird die Reaktion unter atmosphärischem Druck durchgeführt, jedoch ist es auch möglich, unter erhöhtem oder vermindertem Druck zu arbeiten.

Verfahren iii)

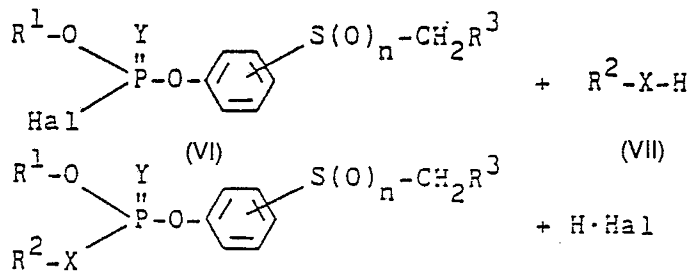

(VI) (VII)

(I)

Zu speziellen Beispielen für das Phosphorsäureesterhalogenid der allgemeinen Formel (VI), die als Ausgangsmaterial bei dem Verfahren, das durch das vorstehende Reaktionsschema dargestellt ist, eingesetzt werden, zählen

O-Ethyl-O-[4-(2,2,2-trifluoroethylthio) phenyl]-phosphatchlorid,
O-Methyl-O-[4-(2,2,2-trifluoroethylthio) phenyl]-phosphatchlorid,
O-Ethyl-O-[4-(2,2,3,3-tetrafluoropropylthio)-phenyl] phosphatchlorid,
O-Ethyl-O-[2-(2,2,2-trifluoroethylthio) phenyl]-phosphatchlorid,
O-Ethyl-O-[2-(2,2,3,3-tetrafluoropropylthio)-phenyl] phosphatchlorid,
O-Ethyl-O-[4-(2,2,2-trifluoroethylsulfonyl)-phenyl] phosphatchlorid,
O-Ethyl-O-[4-(2,2,3,3,3-pentafluoropropylthio)-phenyl] phosphatchlorid, und
O-Ethyl-O-[4-(2,2,3,3-tetrafluoropropylsulfonyl)-phenyl] phosphatchlorid.

Neben den vorstehenden Phosphatchloriden sind die entsprechenden Thiophosphatchloride ebenfalls zu nennen. Auch die entsprechenden Bromide können als Beispiele angeführt werden.

Zu Beispielen für die Verbindung der allgemeinen Formel (VII), die in gleicher Weise Ausgangsmaterialien sind, zählen
Methanol,
Ethanol,
Ethanthiol,
Propanol,
Propan-1-thiol,
Butan-2-thiol,
2-Ethoxyethanthiol und
Isopropylamin.

Ein Beispiel für das vorstehende Verfahren ist im Folgenden dargestellt.

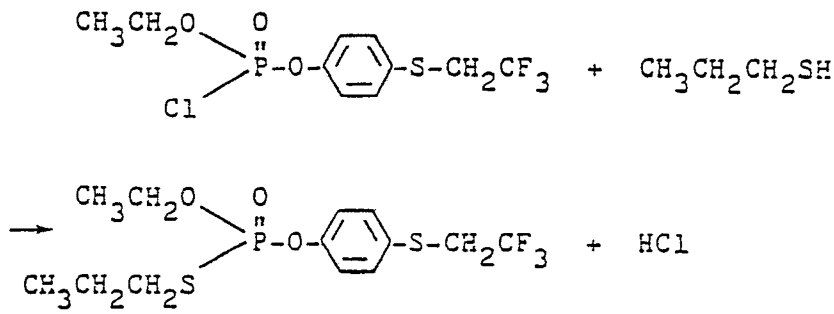

Das vorstehende Verfahren kann bei einer Temperatur innerhalb eines weiten Bereichs unter Verwendung der gleichen inerten Lösungsmittel oder Verdünnungsmittel, wie sie im Vorstehenden beispielhaft angegeben sind, mit Ausnahme von Wasser und Alkoholen, durchgeführt werden. Die Reaktion kann allgemein bei einer Temperatur zwischen etwa − 20 °C und dem Siedepunkt der Mischung, zweckmäßigerweise zwischen etwa 0 °C und etwa 100 °C, durchgeführt werden. Zweckmäßigerweise wird die Reaktion unter atmosphärischem Druck durchgeführt, jedoch ist es auch möglich, unter erhöhtem oder vermindertem Druck zu arbeiten.

Das vorstehende Verfahren kann in Gegenwart der gleichen säurebindenden Mittel, wie sie im Vorstehenden beispielhaft angegeben sind, mit Ausnahme der Alkoholate, durchgeführt werden.

Verfahren iv)

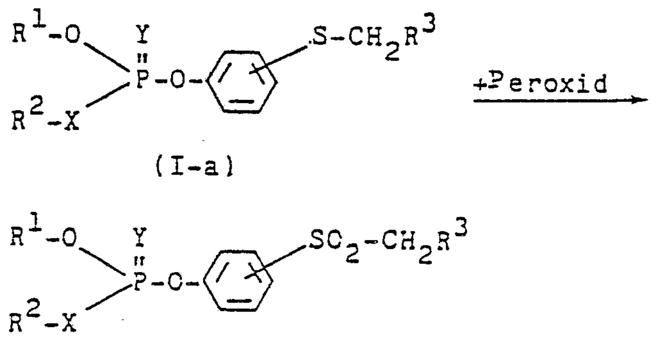


(I-a)

(I-b)

Ein Beispiel hierfür ist O,O-Diethyl-O-[4-(2,2,2-trifluoroethylthio) phenyl] thiophosphat.

In dem vorstehenden Reaktionsschema sind spezielle Beispiele für das Peroxid m-Chloroperoxybenzoesäure und Hydrogenperoxid.

Das vorstehende Verfahren kann bei einer Temperatur innerhalb eines weiten Bereichs unter Verwendung der gleichen inerten Lösungsmittel oder Verdünnungsmittel, wie sie im Vorstehenden beispielhaft angegeben sind, durchgeführt werden. Die Reaktion wird allgemein bei einer Temperatur zwischen etwa − 20 °C und dem Siedepunkt der Mischung, zweckmäßigerweise zwischen etwa 0 °C und etwa 100 °C, durchgeführt. Zweckmäßigerweise wird die Reaktion unter atmosphärischem Druck durchgeführt, jedoch ist es auch möglich, unter erhöhtem oder vermindertem Druck zu arbeiten.

Die Phenol-Derivate der allgemeinen Formel (III) gemäß der vorliegenden Erfindung, die Zwischenprodukte für die Herstellung der Verbindungen der allgemeinen Formel (I) sind, können mittels der nachstehenden Verfahren v) unv vi) hergestellt werden.

Verfahren v) (n = 0)

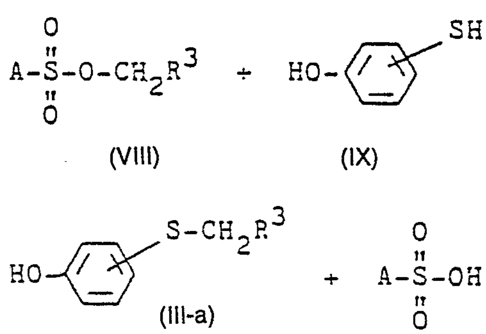

(VIII) (IX)

(III-a)

Zu Beispielen für A zählen nicht nur die gleichen niederen Alkyl-Gruppen, wie sie im Vorstehenden beispielhaft für $R^1$ angegeben sind, sondern auch solche Alkyl-Gruppen wie n-Pentyl, n-Hexyl, n-Heptyl und n-Octyl sowie Aryl-Gruppen wie Phenyl, o-, m- oder p-Tolyl, 2,3-Xylyl, 2,4-Xylyl und 2,5-Xylyl.

Zu speziellen Beispielen für die Verbindung der allgemeinen (VIII), zählen

2,2,2-Trifluoroethyl-methansulfonat,
2,2,2-Trifluoroethyl-p-toluolsulfonat,
2,2,2-Trifluoroethyl-benzolsulfonat,
2,2,3,3-Tetrafluoropropyl-p-toluolsulfonat und
2,2,3,3,3-Pentafluoropropyl-p-toluolsulfonat.

Spezielle Beispiele für das Hydroxyphenylthiol der Formel (IX), sind 2-Hydroxyphenylthiol und 4-Hydroxyphenylthiol.

Das vorerwähnte Herstellungsverfahren wird anhand eines typischen Beispiels im einzelnen erläutert.

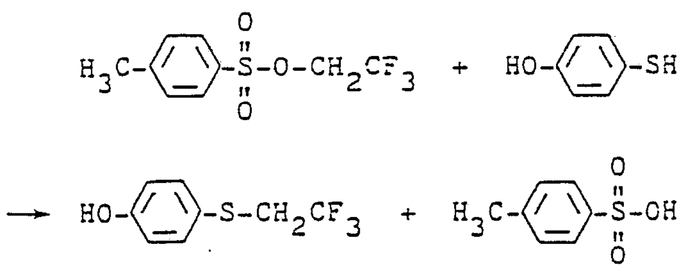

Das vorstehende Verfahren kann in einem aprotischen polaren Lösungsmittel in Gegenwart einer Base wie Natriumcarbonat, Kaliumcarbonat, Natriumhydroxid oder Kaliumhydroxid durchgeführt werden, wobei das angestrebte Produkt in hoher Reinheit und Ausbeute erhalten wird. Beispiele für solche Lösungsmittel sind Dimethylformamid, Dimethylacetamid, Dimethylsulfoxid, Sulfolan und N-Methyl-2-pyrrolidon. Tertiäre Alkohole wie tert-Butanol können verwendet werden, obwohl sie protische polare Lösungsmittel sind.

Das vorstehende Verfahren kann bei einer Temperatur innerhalb eines weiten Bereichs, im allgemeinen bei einer Temperatur zwischen etwa – 20 °C und dem Siedepunkt der Mischung, zweckmäßigerweise zwischen etwa 0 °C und etwa 100 °C, durchgeführt werden. Zweckmäßigerweise wird die Reaktion unter atmosphärischem Druck durchgeführt, jedoch ist es auch möglich, unter erhöhtem oder vermindertem Druck zu arbeiten.

Verfahren vi) (n = 2)

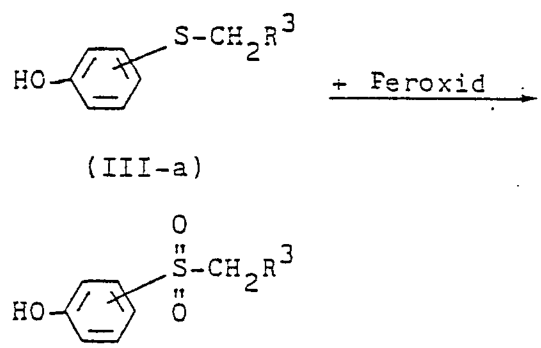

(III-a)

(III-b)

Zu speziellen Beispielen für die Verbindung der allgemeinen Formel (III-a) in dem Reaktionsschema zählen

4-(2,2,2-Trifluoroethylthio) phenol,
2-(2,2,2-Trifluoroethylthio) phenol,
4-(2,2,3,3-Tetrafluoropropylthio) phenol,
2-(2,2,3,3-Tetrafluoropropylthio) phenol und
4-(2,2,3,3,3-Pentafluoropropylthio) phenol.

In dem vorstehenden Reaktionsschema sind spezielle Beispiele für das Peroxid m-Chloroperoxybenzoesäure und Hydrogenperoxid.

Das vorerwähnte Herstellungsverfahren wird anhand eines typischen Beispiels im einzelnen erläutert.

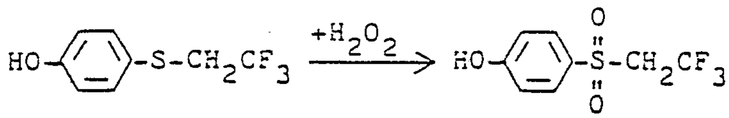


Das vorgenannte Verfahren kann mittels einer für eine Oxidation üblichen Arbeitsweise unter Verwendung der gleichen inerten Lösungsmittel, wie sie im Vorstehenden beschrieben sind, durchgeführt werden. Das vorstehende Verfahren kann bei einer Temperatur innerhalb eines weiten Bereichs, im allgemeinen bei einer Temperatur zwischen etwa – 20 °C und dem Siedepunkt der Mischung, zweckmäßigerweise zwischen etwa 0 °C und etwa 100 °C, durchgeführt werden. Zweckmäßigerweise wird die Reaktion unter atmosphärischem Druck durchgeführt, jedoch ist es auch möglich, unter erhöhtem oder vermindertem Druck zu arbeiten.

In den allgemeinen Formeln bedeutet das Niederalkyl $R^1$ ein geradkekttiges oder kettenverzweigtes Alkyl mit 1 bis 6, bevorzugt 1 bis 4, Kohlenstoff-Atomen, z. B. Methyl, Ethyl, n- und i-Propyl, n-, i-, sec- und tert-Butyl. Vor allen anderen bevorzugt werden Methyl und Ethyl.

In den allgemeinen Formeln bedeutet das Niederalkyl $R^2$ ein geradkettiges oder kettenverzweigtes Alkyl mit 1 bis 6, bevorzugt 1 bis 4, Kohlenstoff-Atomen, z. B. Methyl, Ethyl, n- und i-Propyl, n-, i-, sec- und tert-Butyl. Vor allen anderen bevorzugt werden Methyl, Ethyl, n- und i-Propyl und sec-Butyl.

In den allgemeinen Formeln besteht die Niederalkoxyniederalkyl-Gruppe $R^2$ aus geradkettigen oder verzweigten Alkyl- und Alkoxy-Struktureinheiten und enthält in jeder dieser Alkyl- und Alkoxy-Struktureinheiten 1 bis 6, bevorzugt 1 bis 4, Kohlenstoff-Atome, z. B. Methoxymethyl, Ethoxymethyl, Methoxyethyl, Ethoxyethyl, n- und i-Propoxyethyl und Methoxypropyl.

In den allgemeinen Formeln ist die fluoro-substituierte niedere Alkyl-Gruppe $R^3$ geradkettig oder verzweigt und besitzt 1 bis 6, bevorzugt 1 bis 4, Kohlenstoff-Atome. Diese Gruppe ist durch 1 bis 5, bevorzugt durch 1 bis 4, Fluor-Atome substituiert. Beispiele für diese Gruppe sind die Trifluormethyl-Gruppe und die 1,1-Difluoro-2,2-difluoroethyl-Gruppe.

In den allgemeinen Formeln befindet sich die Gruppe $S(O)_n—CH_2—R^3$ vorzugsweise in der 2- oder 4-Stellung und besonders bevorzugt in der 4-Stellung des Phenoxy-Rings, und n bezeichnet 0 oder 2, vorzugsweise 0.

Bevorzugte Verbindungen der Formel (I) sind Verbindungen, in denen

$R^1$ Alkyl mit 1 bis 4 Kohlenstoff-Atomen bezeichnet,

$R^2$ Alkyl mit 1 bis 4 Kohlenstoff-Atomen oder Alkoxyalkyl mit jeweils1 bis 4 Kohlenstoff-Atomen in der Alkyl- und der Alkoxy-Struktureinheit bezeichnet,

$R^3$ fluorosubstituiertes Alkyl mit 1 bis 4 Kohlenstoff-Atomen, das durch 1 bis 4 Fluor-Atome substituiert ist, bezeichnet,

X für O, S oder NH steht,

Y für S oder O steht und

n für 0 oder 2 steht.

In diesen Verbindungen befindet sich die $S(O)_n—CH_2—R^3$-Gruppe vorzugsweise in der 2- oder 4-Stellung, besonders bevorzugt in der 4-Stellung, des Phenoxy-Rings.

Besonders bevorzugt sind Verbindungen der Formel (I), in denen

$R^1$ Methyl oder Ethyl bezeichnet,

$R^2$ Methyl, Ethyl, n- und i-Propyl, sec-Butyl und Ethoxyethyl bezeichnet,

$R^3$ Trifluoromethyl oder 1,1-Difluoro-2,2-difluoroethyl bezeichnet,

X für O, S oder NH steht,

Y für O oder S steht,

n für 0 oder 2, insbesondere für 0, steht und

die $S(O)_n—CH_2—R^3$-Gruppe sich in der 2- oder 4-Stellung, insbesondere in der 4-Stellung, des Phenoxy-Rings befindet.

Für den Einsatz als insektizides, akazirides oder nematozides Mittel können die Verbindungen gemäß der vorliegenden Erfindung als solche direkt nach dem Verdünnen mit Wasser angewandt oder vorher zu verschiedenen Wirkstoffpräparaten formuliert werden, wie sie unter Verwendung landwirtschaftlich unbedenklicher Hilfsstoffe durch Verfahren gewonnen werden, die bei der praktischen Herstellung von Agrochemikalien allgemein angewandt werden. Im praktischen Gebrauch werden die pestiziden Mittel in Form ihrer verschiedenen Präparate entweder unmittelbar oder nach dem Verdünnen mit Wasser auf die gewünschte Konzentration eingesetzt.

Beispiele für die landwirtschaftlich unbedenklichen Hilfsstoffe, sind Verdünnungsmitel (Lösungsmittel, Streckmittel, Träger), oberflächenaktive Mittel (Lösungsvermittler, Emulgatoren, Dispergiermittel, Netzmittel), Stabilisatoren, Haftmittel, Aerosol-Treibmitel und synergistische Mittel.

Beispiele für die Lösungsmittel sind Wasser und organische Lösungsmittel, beispielsweise Kohlenwasserstoffe [z. B. n-Hexan, Petrolether, Naphtha, Erdöl-Fraktionen (z. B. Paraffinwachse, Kerosin,

Leichtöle, Mittelöle, Schweröle), Benzol, Toluol und Xylole], halogenierte Kohlenwasserstoffe (z. B. Methylenchlorid, Kohlenstofftetrachlorid, Trichloroethylen, Ethylenchlorid, Ethylendibromid, Chlorbenzol und Chloroform), Alkohole (z. B. Methylalkohol, Ethylalkohol, Propylalkohol und Ethylenglycol), Ether (z. B. Ethylether, Ethylenoxid und Dioxan), Alkohol-ether (z. B. Ethylenglycol-monomethylether), Ketone (z. B. Aceton und Isophoron), Ester (z. B. Ethylacetat und Amylacetat), Amide (z. B. Dimethylformamid und Dimethylacetamid) und Sulfoxide (z. B. Dimethylsulfoxid).

Beispiele für die Streckmittel oder Träger umfassen anorganische Pulver, beispielsweise Löschkalk, Magnesiumkalk, Gips, Calciumcarbonat, Siliciumdioxid, Perlit, Bimsstein, Calcit, Diatomeenerde, amorphes Siliciumdioxid, Aluminiumoxid, Zeolithe und Tonminerale (z. B. Pyrophyllit, Talkum, Montmorillonit, Beidellit, Vermikulit, Kaolinit und Glimmer), pflanzliche Pulver wie beispielsweise Getreidepulver, Stärkearten, verarbeitete Stärkearten, Zucker, Glucose und zerkleinerte Stengel von Pflanzen, sowie Pulver aus synthetischen Harzen wie Phenol-Harzen, Harnstoff-Harzen und Vinylchlorid-Harzen.

Beispiele für die oberflächenaktiven Mittel umfassen anionische oberflächenaktive Mittel wie Alkylschwefelsäureester (z. B. Natriumlaurylsulfat), Arylsulfonsäure-Salze (z. B. Alkylarylsulfonsäure-Salze und Natriumalkylnaphthalinsulfonate), Bernsteinsäure-Salze und Salze von Schwefelsäureestern von Polyethylenglycol-alkylarylethern, kationische oberflächenaktive Mittel wie Alkylamine (z. B. Laurylamin, Stearyltrimethylammoniumchlorid und Alkyldimethylbenzylammoniumchlorid) und Polyoxyethylenalkylamine, nicht-ionische oberflächenaktive Mittel wie Polyoxyethylenglycolether (z. B. Polyoxyethylenalkylarylether und deren Kondensationsprodukte), Polyoxyethylenglycolester (z. B. Polyoxyethylenfettsäureester) und Ester mehrwertiger Alkohole (z. B. Polyoxyethylensorbitan-monolaurat) sowie amphotere oberflächenaktive Mittel.

Beispiele für andere Hilfsstoffe umfassen Stabilisatoren, Haftmittel (z. B. landwirtschaftliche Seifen, Casein-Kalk, Natriumalginat, Polyvinylalkohol, Haftmittel vom Vinylacetat-Typ und Acryl-Haftmittel), Aerosol-Treibmittel (z. B. Trichlorofluoromethan, Dichlorofluoromethan, 1,2,2-Trichloro-1,1,2-trifluoroethan, Chlorbenzol, verflüssigtes Erdgas und niedere Ether), die Verbrennung steuernde Mittel für Räucherung und Begasung (z. B. Nitrite, Zink-Pulver und Dicyandiamid), sauerstoff-abgebende Mittel (z. B. Chlorate und Dichromate), die Phytotoxizität senkende Mittel (z. B. Zinksulfat, Eisen(II)-chlorid und Kupfersulfat), wirkungsverlängernde Mittel, Dispersions-Stabilisatoren (z. B. Casein, Tragant, Carboxymethylcellulose und Polyvinylalkohol) und synergistische Mittel.

Die Verbindungen der vorliegenden Erfindung können mittels der allgemein auf dem Gebiet der Agrochemikalien gebräuchlichen Verfahren zu verschiedenen Präparaten formuliert werden. Beispiele für solche Anwendungsformen sind emulgierbare Konzentrate, Öle, benetzbare Pulver, lösliche Pulver, Suspensionen, Stäubemittel, Granulat, pulvrige Mittel, Räuchermittel, Tabletten, Pasten und Kapseln.

Das insektizide, akarizide oder nematozide Mittel gemäß der vorliegenden Erfindung kann etwa 0,1 bis etwa 95 Gew.-%, vorzugsweise etwa von 0,5 bis 90 Gew.-% des vorerwähnten Wirkstoffes enthalten.

Für den praktischen Gebrauch beträgt die geeignete Menge der aktiven Verbindung in den vorgenannten Mitteln in ihren verschiedenen Formen und gebrauchsfertigen Präparaten beispielsweise etwa 0,000 1 bis etwa 20 Gew.-%, vorzugsweise etwa 0,005 bis etwa 10 Gew.-%.

Der Gehalt des Wirkstoffs kann in geeigneter Weise je nach der Form des Präparats oder Mittels, dem Verfahren, Zweck, der Zeit und dem Ort seiner Anwendung, dem Zustand des Auftretens der zu bekämpfenden Schad-Organismen variiert werden.

Erforderlichenfalls können die Verbindungen gemäß der vorliegenden Erfindung weiterhin auch in Kombination mit anderen Agrochemikalien verwendet werden, beispielsweise mit anderen Insektiziden, Fungiziden, anderen Akariziden, anderen Nematoziden, Anti-Virus-Mitteln, Herbiziden, Pflanzen-Wachstumsregulatoren und Lockstoffen [Organophosphorester-Verbindungen, Carbamat-Verbindungen, Dithio (oder thiol) carbamat-Verbindungen, Organochlor-Verbindungen, Dinitro-Verbindungen, organischen Schwefel- oder Metall-Verbindungen, Antibiotika, substituierten Diphenylether-Verbindungen, Harnstoff-Verbindungen und Triazin-Verbindungen] und/oder Düngemitteln.

Den im Vorstehenden bezeichneten Wirkstoff enthaltende verschiedenartige Mittel und gebrauchsfertige Präparate können mittels verschiedener Verfahren zur Anwendung gebracht werden, wie sie allgemein für das Aufbringen von Agrochemikalien gebräuchlich sind, beispielsweise durch Dispergieren (Versprühen von Flüssigkeiten, Vernebeln, Zerstäuben, Stäuben, Streuen von Granulat, Wasser-Oberflächenbehandlung und Gießen), Räuchern und Begasen, Bodenbehandlung (Vermischen mit dem Boden, Streuen, Spritzen, Bedampfen und Einspritzen), Oberflächen-Anwendung (z. B. Beschichten, Aufbringen in Form von Bändern, Beschichten mit Staub und Bedecken) und Eintauchen. Sie können auch mittels des sogenannten Ultra-Low-Volume-Sprühverfahrens aufgebracht werden. Nach diesem Verfahren kann der Wirkstoff sogar in einer Konzentration von 100 % zur Anwendung gelangen.

Die Aufwandmengen pro Flächeneinheit beträgt etwa 0,03 bis etwa 10 kg/ha, vorzugsweise etwa 0,3 bis etwa 6 kg/ha. In besonders gelagerten Fällen können oder sollten sogar die Aufwandmengen außerhalb des angegebenen Bereichs liegen.

Gemäß der vorliegenden Erfindung wird ein insektizides, akarizides oder nematozides Mittel zur Verfügung gestellt, das als Wirkstoff die Verbindung der allgemeinen Formel (I) sowie ein Verdünnungsmittel (ein Lösungsmittel und/oder ein Streckmittel und/oder einen Träger) und/oder ein oberflächenaktives Mittel und, falls weiterhin erforderlich, einen Stabilisator, ein Haftmittel, ein synergistisches Mittel enthält.

Die vorliegende Erfindung macht weiterhin ein Verfahren zur Bekämpfung von Insekten, Milben und Nematoden verfügbar, das das darin besteht, daß eine Verbindung der allgemeinen Formel (I) allein oder in Form einer Mischung mit einem Verdünnungsmittel (einem Lösungsmittel und/oder einem Streckmittel und/oder einem Träger) und/oder einem oberflächenaktiven Mittel und, falls weiterhin erforderlich, einem Stabilisator, einem Haftmittel, einem synergistischen Mittel etc. auf Insekten, Milben und Nematoden und/oder ihren Lebensraum oder den Ort, an dem mit dem Auftreten solcher Schädlinge zu rechnen ist, aufgebracht wird.

Die vorliegende Erfindung wird durch die folgenden Beispiele näher erläutert.

Beispiel 1

Benetzbares Pulver.

15 Teile der Verbindung Nr. 1 der Erfindung, 80 Teile eines Gemisches (1 : 5) aus Weißruß (feinpulvrigem wasserhaltigen amorphen Siliciumoxid) und Tonpulver, 2 Teile Natriumalkylbenzolsulfonat und 3 Teile Natriumalkylnaphthalinsulfonat/Formaldehyd-Kondensat werden pulverisiert und zu einem benetzbaren Pulver vermischt. Dieses wird mit Wasser verdünnt und auf Insekten, Milben oder Nematoden und/oder ihren Lebensraum oder den Ort, an dem mit dem Auftreten solcher Schädlinge zu rechnen ist, aufgesprüht.

Beispiel 2

Emulgierbares Konzentrat.

30 Teile der Verbindung Nr. 2 der Erfindung, 55 Teile Xylol, 8 Teile Polyoxyethylen-alkylphenylether und 7 Teile Calciumalkylbenzolsulfonat werden unter Rühren miteinander vermischt, wodurch ein emulgierbares Konzentrat hergestellt wird. Dieses wird mit Wasser verdünnt und auf Insekten, Milben oder Nematoden und/oder ihren Lebensraum oder den Ort, an dem mit dem Auftreten solcher Schädlinge zu rechnen ist, aufgesprüht.

Beispiel 3

Stäubemittel.

Die Verbindung Nr. 3 der Erfindung (2 Teile) und 98 Teile Tonpulver wurden pulverisiert und gemischt, wodurch ein Stäubemittel hergestellt wird. Dieses wird auf Insekten, Milben oder Nematoden und/oder ihren Lebensraum oder den Ort, an dem mit dem Auftreten solcher Schädlinge zu rechnen ist, aufgestreut.

Beispiel 4

Stäubemittel.

Die Verbindung Nr. 4 der Erfindung (1,5 Teile), 0,5 Teile Isopropylhydrogenphosphat (PAP) und 98 Teile Tonpulver wurden pulverisiert und gemischt, wodurch ein Stäubemittel hergestellt wurde. Dieses wird auf Insekten, Milben oder Nematoden und/oder ihren Lebensraum oder den Ort, an dem mit dem Auftreten solcher Schädlinge zu rechnen ist, aufgestreut.

Beispiel 5

Granulat.

Wasser (25 Teile) wird zu einer Mischung aus 10 Teilen der Verbindung Nr. 5 der Erfindung, 30 Teilen Bentonit (Montmorillonit), 58 Teilen Talkum und 2 Teilen Ligninsulfonat zugesetzt, und das Gemisch wird gut geknetet. Die Mischung wird mittels eines Extruder-Granulators zu einem Granulat mit einer Korngröße von 0,43 bis 2,0 mm (10 bis 40 mesh) verarbeitet und bei 40 °C bis 50 °C getrocknet, wodurch ein Granulat gebildet wird. Das Granulat wird auf Insekten, Milben oder Nematoden und/oder ihren Lebensraum oder den Ort, an dem mit dem Auftreten solcher Schädlinge zu rechnen ist, aufgestreut.

Beispiel 6

Granulat.

Ein Drehmischer wird mit 95 Teilen Tonmineral-Teilchen mit einer Teilchengrößen-Verteilung zwischen 0,2 und 2 mm beschickt, und unter Drehen des Mischers werden 5 Gew.-Teile der öligen Verbindung Nr. 6 der Erfindung zur gleichmäßigen Benetzung unter Bildung eines Granulats auf die

Tonmineral-Teilchen aufgesprüht. Dieses wird auf Insekten, Milben oder Nematoden und/oder ihren Lebensraum oder den Ort, an dem mit dem Auftreten solcher Schädlinge zu rechnen ist, aufgebracht.

Beispiel 7

Öl.

Die Verbindung Nr. 7 der Verbindung (0,5 Teile) und 99,5 Teile Kerosin werden unter Rühren miteinander vermischt, wodurch ein Öl gebildet wird. Dieses wird auf Insekten, Milben oder Nematoden und/oder ihren Lebensraum oder den Ort, an dem mit dem Auftreten solcher Schädlinge zu rechnen ist, aufgesprüht.

Die unerwartete Überlegenheit und die ausgeprägten Wirkungen der Verbindungen gemäß der vorliegenden Erfindung sind aus den Ergebnissen der im Folgenden beschriebenen Tests zu entnehmen, in denen diese Verbindungen gegen verschiedenartige Schädlinge, darunter Insekten, Milben und Nematoden, eingesetzt wurden.

Test-Beispiel 1

Test mit Larven von Spodoptera litura :

Herstellung eines Test-Präparats :

| | | |
|---|---|---|
| Lösungsmittel : Xylol | | 3 Gew.-Teile |
| Emulgator : | Polyoxyethylen-alkylphenylether | 1 Gew.-Teil |

Ein Test-Präparat wurde hergestellt durch Vermischen von 1 Gew.-Teil der betreffenden aktiven Verbindungen mit dem bezeichneten Lösungsmitel und dem Emulgator in den angegebenen Mengen und Verdünnen mit Wasser auf eine vorher festgesetzte Konzentration verdünnt.

Test-Verfahren :

Blätter süßer Kartoffeln wurden in die wäßrige Verdünnung mit der vorbestimmten Wirkstoff-Konzentration jeder der in der Tabelle 1 angegebenen aktiven Verbindungen getaucht. Nach dem Trocknen an der Luft wurden die Blätter der süßen Kartoffeln in Petrischalen von 9 cm Durchmesser gelegt. 10 Larven im 3. Stadium von Spodoptera litura wurden in jede Schale eingesetzt. Die Petrischalen wurden in einen Raum mit konstanter Temperatur von 28 °C gestellt. Nach 24 h wurde die Zahl der toten Insekten bestimmt und das Vernichtungsverhältnis berechnet.

Die Ergebnisse sind in der Tabelle 1 aufgeführt.

Tabelle 1

| Verbindung Nr. | Wirkstoff-Konzentration ppm | Vernichtungs-verhältnis % |
|---|---|---|
| 1 | 10 | 100 |
| 3 | 10 | 100 |
| 4 | 10 | 100 |
| 5 | 10 | 100 |
| 8 | 10 | 100 |
| 9 | 10 | 100 |
| Vergleichs-Verbindung Nr. | | |
| X - 1 | 100 | 20 |
| XI - 1 | 100 | 90 |
| A | 100 | 50 |

Anmerkungen zu Tabelle 1 :

1. Die Verbindungs-Nummern entsprechen denjenigen der Synthesebeispiele und der Tabelle 10.
2. Die Vergleichs-Verbindungen sind die nachstehenden : Vergleichs-Verbindung X-1 :

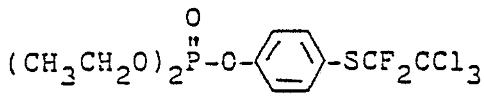

(die in der US-PS-3 755 511 beschriebene Verbindung) ;

Vergleichs-Verbindung XI-1 :

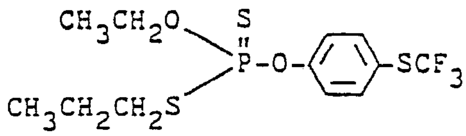

(die in der US-PS-4 139 615 beschriebene Verbindung) ;

Vergleichs-Verbindung A :

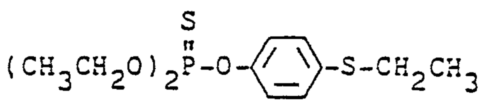

(die in der JP-Patentveröffentlichung Nr. 778/1960 beschriebene Verbindung).

Test-Beispiel 2

Test mit Callosobruchus chinensis :

Test-Verfahren :

Ein Filterpapier wurde jeweils auf dem Boden einer Petrischale von 9 cm Durchmesser ausgebreitet, und 1 ml einer wäßrigen Verdünnung jeder der in der Tabelle 2 aufgeführten aktiven Verbindungen mit einer vorbestimmten Konzentration, die wie in Test-Beispiel 1 hergestellt worden war, wurde jeweils in einer Schale gegeben. 20 Exemplare von Callosobruchus chinensis wurden eingesetzt, und die Petrischalen wurden in einen Raum mit einer konstanten Temperatur von 28 °C gestellt. Nach 24 h wurde die Zahl der toten Insekten bestimmt und das Vernichtungsverhältnis berechnet.

Die Ergebnisse sind in der Tabelle 2 aufgeführt.

Tabelle 2

| Verbindung Nr. | Wirkstoff-Konzentration ppm | Vernichtungs-verhältnis % |
|---|---|---|
| 2 | 1 | 100 |
| 3 | 10 | 100 |
| 4 | 10 | 100 |
| 5 | 10 | 100 |
| 6 | 1 | 100 |
| Vergleichs-Verbindung Nr. | | |
| X - 1 | 10 | 0 |
| XI - 1 | 10 | 60 |
| A | 10 | 80 |

Anmerkungen zu Tabelle 2 :

1. Die Verbindungs-Nummern entsprechen denjenigen der Synthesebeispiele und der Tabelle 10.
2. Die Vergleichs-Verbindungen sind die gleichen wie in der Tabelle 1.

Test-Beispiel 3

Test mit gegen Organophosphor-Insektizide resistenten Nephotettix cincticeps :

Test-Verfahren :

Auf Reispflanzen von etwa 10 cm Höhe, die jeweils in einen Topf von 12 cm Durchmesser gepflanzt waren, wurden jeweils 10 ml pro Topf einer wäßrigen Verdünnung einer der in der Tabelle 3 aufgeführten aktiven Verbindungen mit einer vorbestimmten Konzentration, die wie in Test-Beispiel 1 hergestellt wurde, aufgesprüht. Nach dem Trocknen der Reispflanzen wurde jeder Topf mit einem Drahtkorb von 7 cm Durchmesser und 14 cm Höhe bedeckt, und 30 ausgewachsene weibliche Exemplare von Nephotettix cincticeps, die gegen Organophosphat-Mittel resistent waren, wurden in dem drahtkorb ausgesetzt. Nach 24-stündiger Aufbewahrung in einem Raum konstanter Temperatur wurde die Zahl der toten Insekten bestimmt und das Vernichtungsverhältnis berechnet.

Die Ergebnisse sind in der Tabelle 3 aufgeführt.

Tabelle 3

| Verbindung Nr. | Wirkstoff-Konzentration ppm | Vernichtungs-verhältnis % |
|---|---|---|
| 3 | 10 | 100 |
| 4 | 10 | 100 |
| 8 | 100 | 100 |
| 9 | 100 | 100 |
| 13 | 100 | 100 |
| Vergleichs-Verbindung Nr. | | |
| X - 1 | 100 | 50 |
| XI - 1 | 100 | 0 |
| A | 100 | 0 |

Anmerkungen zu Tabelle 3 :
1. Die Verbindungs-Nummern entsprechen denjenigen der Tabelle 10.
2. Die Vergleichs-Verbindungen sind die gleichen wie in der Tabelle 1.

Test-Beispiel 4

Test mit Musca domestica :

Test-Verfahren :

Ein Filterpapier wurde jeweils auf dem Boden einer Petrischale von 9 cm Durchmesser ausgebreitet, und jeweils 1 ml einer wäßrigen Verdünnung jeder der in der Tabelle 4 aufgeführten aktiven Verbindungen mit einer vorbestimmten Konzentration, wie in Testbeispiel 1 hergestellt wurde, wurde in eine Schale gegeben. 10 ausgewachsene weibliche Exemplare von Musca domestica, die gegen handelsübliche Organophosphat-Mittel resistent waren, wurden in die Schale eingesetzt. Die Schalen wurden in einem Raum mit einer konstanten Temperatur von 29 °C gestellt. Nach 24 h wurde die Zahl der toten Insekten bestimmt und das Vernichtungsverhältnis berechnet.

Die Ergebnisse sind in der Tabelle 4 aufgeführt.

(Siehe Tabelle 4, Seite 18 f.)

Tabelle 4

| Verbindung Nr. | Wirkstoff-Konzentration ppm | Vernichtungs-verhältnis % |
|---|---|---|
| 3 | 100 | 100 |
| 4 | 100 | 100 |
| Vergleichs-Verbindung Nr. | | |
| X - 1 | 100 | 0 |
| XI - 1 | 100 | 0 |
| A | 100 | 0 |

Anmerkungen zu Tabelle 4 :
1. Die Verbindungs-Nummern entsprechen denjenigen der Tabelle 10.
2. Die Vergleichs-Verbindungen sind die gleichen wie in der Tabelle 1.

Test-Beispiel 5

Test mit Tetranychus telarius :

Test-Verfahren :

50 bis 100 ausgewachsene Exemplare von Tetranychus telarius, die gegen Organophosphat-Mittel resistent waren, wurden auf den Blättern von Vits-Bohnen (Phaseolus vulgaris Linn.) im Stadium der Entwicklung von 2 Haupt-Blättern ausgesetzt, die in Töpfen mit einem Durchmesser von 6 cm gezogen worden waren. Nach 2 Tagen wurde eine wäßrige Verdünnung jeder der in der Tabelle 5 aufgeführten aktiven Verbindungen mit einer vorbestimmten Konzentration, die wie in Test-Beispiel 1 hergestellt wurde, in einer Menge von 40 ml je Topf gesprüht. Die Töpfe wurden in ein Gewächshaus gestellt, und nach 10 Tagen wurde die Bekämpfungswirkung mit Hilfe der nachstehend bezeichneten Indices bewertet :

3 : Anteil der lebenden ausgewachsenen Insekten : 0 % ;

2 : Anteil der lebenden ausgewachsenen Insekten : mehr als 0 % und weniger als 5 %, bezogen auf eine unbehandelte Fläche ;

1 : Anteil der lebenden ausgewachsenen Insekten : 5 bis 50 %, bezogen auf die unbehandelte Fläche ;

0 : Anteil der lebenden ausgewachsenen Insekten : mehr als 50 %, bezogen auf die unbehandelte Fläche.

Die Ergebnisse sind in der Tabelle 5 aufgeführt.

(Siehe Tabelle 5 Seite 19 f.)

Tabelle 5

| Verbindung Nr. | Wirkstoff-Konzentration ppm | Bekämpfungs-Index |
|---|---|---|
| 1 | 100 | 3 |
| 3 | 100 | 3 |
| 4 | 100 | 3 |
| 5 | 100 | 3 |
| 8 | 100 | 3 |
| 9 | 100 | 3 |
| 12 | 100 | 3 |
| 13 | 100 | 3 |
| Vergleichs-Verbindung Nr. | | |
| X - 1 | 100 | 0 |
| XI - 1 | 100 | 0 |
| A | 100 | 0 |

Anmerkungen zu Tabelle 5 :
1. Die Verbindungs-Nummern entsprechen denjenigen der Synthesebeispiele und der Tabelle 10.
2. Die Vergleichs-Verbindungen sind die gleichen wie in der Tabelle 1.

Test-Beispiel 6

Test mit Blatella Germanica

Test-Verfahren :

Ein Filterpapier wurde jeweils auf dem Boden einer Petrischale von 9 cm Durchmesser gelegt, und 1 ml einer wäßrigen Verdünnung jeder in der Tabelle 6 aufgeführten aktiven Verbindungen mit einer vorbestimmten Konzentration, die wie in Test-Beispiel 1 hergestellt wurde, wurde hinzugegeben. 10 ausgewachsene Exemplare von Blatella Germanica wurden in die Schale eingesetzt. Die Schalen wurden in einen Raum mit einer konstanten Temperatur von 28 °C gestellt. Nach 24 h wurde die Zahl der toten Insekten bestimmt und das Vernichtungsverhältnis berechnet.

Die Ergebnisse sind in der Tabelle 6 aufgeführt.

(Siehe Tabelle 6, Seite 20 f.)

Tabelle 6

| Verbindung Nr. | Wirkstoff-Konzentration ppm | Vernichtungs-verhältnis % |
|---|---|---|
| 2 | 10 | 100 |
| 4 | 100 | 100 |
| 6 | 10 | 100 |
| 7 | 100 | 100 |
| 10 | 100 | 100 |
| 11 | 100 | 100 |
| Vergleichs-Verbindung Nr. | | |
| X - 1 | 100 | 80 |
| XI - 1 | 100 | 10 |
| A | 100 | 0 |

Anmerkungen zu Tabelle 6 :
1. Die Verbindungs-Nummern entsprechen denjenigen der Synthesebeispiele und der Tabelle 10.
2. Die Vergleichs-Verbindungen sind die gleichen wie in der Tabelle 1.

Test-Beispiel 7

Test mit Larven von Culex pipiens :

Test-Verfahren :

100 ml einer wäßrigen Verdünnung jeder der in der Tabelle 7 aufgeführten aktiven Verbindungen mit einer vorbestimmten Konzentration, die wie in Testbeispiel 1 hergestellt wurde, wurden jeweils in eine tiefe Petrischale mit einem Durchmesser von 9 cm gegeben, und 25 Larven im 4. Stadium von Culex pipiens wurden in die Schale eingesetzt. Die Schale wurde in einen Raum mit konstanter Temperatur von 28 °C gestellt. Nach 24 h wurde die Zahl der toten Insekten bestimmt und das Vernichtungsverhältnis berechnet.

Die Ergebnisse sind in der Tabelle 7 aufgeführt.

Tabelle 7

| Verbindung Nr. | Wirkstoff-Konzentration ppm | Vernichtungs-verhältnis % |
|---|---|---|
| 1 | 0,1 | 100 |
| 2 | 0,001 | 100 |
| 3 | 0,1 | 100 |
| 4 | 0,01 | 100 |
| 6 | 0,01 | 100 |
| 7 | 0,1 | 100 |
| 9 | 0,01 | 100 |

Tabelle 7 (Fortsetzung)

| Verbindung Nr. | Wirkstoff-Konzentration ppm | Vernichtungsverhältnis % |
|---|---|---|
| 10 | 0,01 | 100 |
| 11 | 0,1 | 100 |
| 12 | 0,1 | 100 |
| 13 | 0,01 | 100 |
| Vergleichs-Verbindung Nr. | | |
| X - 1 | 0,1 | 40 |
| XI - 1 | 0,1 | 70 |
| A | 0,1 | 70 |

Anmerkungen zu Tabelle 7 :
1. Die Verbindungs-Nummern entsprechen denjenigen der Synthesebeispiele und der Tabelle 10.
2. Die Vergleichs-Verbindungen sind die gleichen wie in der Tabelle 1.

Test-Beispiel 8

Test mit Meloidogyne incognita :

Herstellung des Test-Präparats :

2 Teile jeder der aktiven Verbindungen, die in der Tabelle 8 aufgeführt sind, und 98 Teile Talkum wurden pulverisiert und miteinander vermischt.

Test-Verfahren :

Das wie im Vorstehenden beschrieben hergestellte Test-Präparat wurde zu durch Meloidogyne incognita verseuchter Erde in solchen Mengen hinzugefügt, daß es in Konzentrationen von 50, 25, 10 und 5 ppm vorlag. Erde und Präparat wurden unter Rühren gleichmäßig miteinander vermischt und anschließend in Töpfe von 2 $dm^2$ gefüllt. In diese Mischung wurden etwa 20 Körner Tomaten-Samen (Varietät : Kurihara) pro Topf eingesät. Die Tomatenpflanzen wurden im Gewächshaus angezogen. Nach vier Wochen wurden die Tomatenpflanzen herausgezogen, ohne dabei ihre Wurzeln zu beschädigen, und der Grad der Schädigung von 10 Wurzeln der Tomatenpflanzen wurde zur Bestimmung des Wurzelgallen-Index nach dem folgenden Maßstab bewertet :

Grad der Schädigung :
0 : keine Gallen-Bildung (vollständige Bekämpfung) ;
1 : leichte Gallen-Bildung ;
3 : starke Gallen-Bildung ;
4 : sehr starke Gallen-Bildung (entsprechend derjenigen unbehandelter Pflanzen).

$$\text{Wurzelgallen-Index} = \frac{\sum \left(\text{Klassen-bewertung} \times \text{Zahl der Individuen}\right)}{\left(\text{Gesamtzahl der bewerteten Individuen}\right) \times 4} \times 100$$

Hieraus wurde die Bekämpfungswirkung mit Hilfe der nachstehenden Formel bestimmt :

$$\text{Bekämpfungswirkung} = \frac{(\text{Wurzelgallen-Index der unbehandelt. Fläche} - \text{Wurzelgallen-Index der behandelt. Fläche})}{\text{Wurzelgallen-Index der unbehandelten Fläche}} \times 100$$

Eine Bekämpfungswirkung von 100 % bezeichnet eine vollständige Bekämpfung. Die Ergebnisse sind in der Tabelle 8 aufgeführt.

Tabelle 8

| Verbindung Nr. | Wirkstoff-Konzentration ppm | Bekämpfungswirkung % |
|---|---|---|
| 3 | 10 | 100 |
| 5 | 25 | 100 |
| 6 | 25 | 100 |
| Vergleichs-Verbindung Nr. | | |
| A | 50 | 0 |

Anmerkungen zu Tabelle 8 :

1. Die Verbindungs-Nummern entsprechen denjenigen der Tabelle 10.
2. Die Vergleichs-Verbindungen sind die gleichen wie in der Tabelle 1.

Die folgenden Synthese-Beispiele beschreiben speziell ein Verfahren zur Herstellung einer Verbindung der allgemeinen Formel (III), die ein Zwischenprodukt für die Herstellung der Verbindung gemäß der vorliegenden Erfindung ist.

Synthese-Beispiel 1

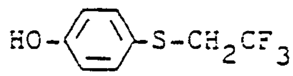

(Verbindung Nr. III-1)

Unter Rühren in einer Stickstoff-Atmosphäre wurden 110 g wasserfreies Kaliumcarbonat zu einer Lösung von 51 g 4-Hydroxyphenylthiol in 250 ml Dimethylformamid hinzugefügt. Weiterhin wurden 112 g 2,2,2-Trifluoroethyl-p-toluolsulfonat bei einer Tempeatur von 25 °C bis 30 °C zugegeben. Die Mischung wurde 3 h bei der selben Temperatur ; anschließend 4 h bei 30 °C bis 40 °C und schließlich 6 h bei 50 °C gerührt. Nach dem Abkühlen wurde die Lösung in 2 l Wasser gegossen, und konzentrierte Salzsäure wurde bei einer Temperatur unterhalb von 20 °C zugesetzt, um den pH der Lösung auf 3 einzustellen. Das erhaltene ölige Produkt wurde mit Toluol extrahiert. Die Toluol-Schicht wurde mit Wasser gewaschen und zusammen mit 600 ml einer 10-proz. wäßrigen Kaliumhydroxid-Lösung gerührt. Die wäßrige Schicht wurde abgetrennt, und Salzsäure wurde bei einer Temperatur unterhalb von 20 °C zugesetzt, um den pH der Lösung auf 2 einzustellen. Das erhaltene ölige Produkt wurde mit Toluol extrahiert. Die Toluol-Schicht wurde abgetrennt, mit Wasser gewaschen und über wasserfreiem Natriumsulfat getrocknet. Das Toluol wurde abgedampft, und der Rückstand wurde unter vermindertem Druck destilliert, wonach 68 g des angestrebten 4-(2,2,2-Trifluoroethylthio) phenols erhalten wurden ; Sdp. 102-105 °C/1,07 mbar (0,8 mm Hg) erhalten wurden.

Analog wurden die durch die allgemeine Formel (III) dargestellten Phenol-Derivate der vorliegenden Erfindung, die in der Tabelle 9 aufgeführt sind, hergestellt.

Tabelle 9

| Verbindung Nr. | Ausgangsmaterial | Ausgangsmaterial | Produkt der allgemeinen Formel (III) |
|---|---|---|---|
| III-2 | 2,2,2-Trifluoroethyl-p-toluolsulfonat | 2-Hydroxyphenylthiol | 2-(2,2,2-Trifluoroethylthio)phenol (63-65°C/0,93 mbar (0,7 mmHg)) |
| III-3 | 2,2,3,3-Tetrafluoropropyl-p-toluolsulfonat | 4-Hydroxyphenylthiol | 4-(2,2,3,3-Tetrafluoropropylthio)phenol (117-119°C/0,67 mbar 0,5 mm Hg)) |
| III-4 | 2,2,3,3-Tetrafluoropropyl-p-toluolsulfonat | 2-Hydroxyphenylthiol | 2-(2,2,3,3-Tetrafluoropropylthio)phenol |
| III-5 | 2,2,3,3,3-Pentafluoropropyl-p-toluolsulfonat | 4-Hydroxyphenylthiol | 4-(2,2,3,3,3-Pentafluoropropylthio)-phenol |

Die folgenden Synthese-Beispiele erläutern speziell das Verfahren zur Herstellung der Organophosphat-Derivate der allgemeinen Formel (I) der vorliegenden Erfindung, die die aktiven Verbindungen gemäß der vorliegenden Erfindung sind.

Synthese-Beispiel 2

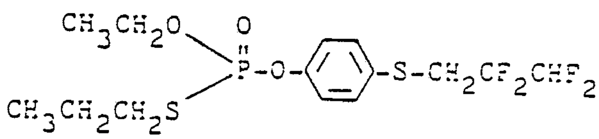

(Verbindung Nr. 1)

4 g 4-(2,2,3,3-Tetrafluoropropylthio) phenol und 1,5 g Triethylamin wurden in 60 ml Toluol gelöst. Zu der Lösung wurden tropfenweise 3,56 g O-Ethyl-S-propylthiophosphatchlorid bei 10 °C bis 20 °C hinzugefügt. Die Mischung wurde 6 h bei 50 °C gerührt. Die Reaktionsmischung wurde abgekühlt, nacheinander mit 2-proz. Salzsäure, einer 2-proz. wäßrigen kaliumhydroxid-Lösung und Wasser gewaschen und über wasserfreiem Natriumsulfat getrocknet. Das Toluol wurde unter vermindertem Druck abdestilliert, wonach durch Destillation des Rückstandes unter vermindertem Druck 3 g des angestrebten O-Ethyl-S-propyl-O-[4-(2,2,3,3-tetrafluoropropylthio) phenyl]-thiophosphats erhalten wurden ; Sdp. 156-158 °C/0,27 mbar (0,2 mm Hg) ; $n_D^{20}$ = 1,504 1.

Synthese-Beispiel 3

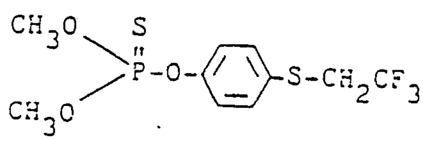

(Verbindung Nr. 2)

Zu einer Mischung aus 4,16 g 4-(2,2,2-Trifluoroethylthio)phenol, 2,8 g wasserfreiem Kaliumcarbonat und 50 ml Methylethylketon wurden tropfenweise 3,17 g O,O-Dimethylthiophosphatchlorid bei 20 °C bis 25 °C unter Rühren hinzugefügt. Die Lösung wurde 6 h bei 50 °C bis 55 °C gerührt, und das

Methylethylketon wurde unter vermindertem Druck abgedampft. Toluol (80 ml) und 80 ml Wasser wurden zugegeben. Die Toluol-Schicht wurde abgetrennt, nacheinander mit einer 2-proz. wäßrigen kaliumhydroxid-Lösung und mit Wasser gewaschen und über wasserfreiem Natriumsulfat getrocknet. Das Toluol und niedrig siedende Substanzen wurden unter vermindertem Druck abgedampft, wonach 5 g des angestrebten O,O-Dimethyl-O-[4-(2,2,2-trifluoroethylthio) phenyl] thiophosphats erhalten wurden ; $n_D^{20} = 1{,}516\,3$.

Analog den Synthese-Beispielen 2 und 3 wurden die durch allgemeine Formel (I) dargestellten Organophosphat-Derivate, die in der Tabelle 10 aufgeführt sind, hergestellt.

(Siehe Tabelle 10, Seite 25 f.)

Tabelle 10

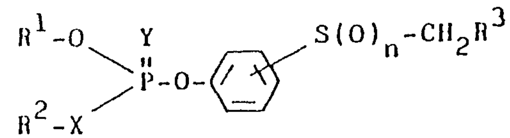

| Verbindung Nr. | X | Y | $R^1$ | $R^2$ | $R^3$ | n | Bindungsposition | Physikalische Konstante |
|---|---|---|---|---|---|---|---|---|
| 3 | S | O | $-C_2H_5$ | $n-C_3H_7$ | $-CF_3$ | 0 | 4- | Sdp. 157-160°C/1,07 mbar (0,8 mm Hg) $n_D^{20}$ 1.5050 |
| 4 | S | O | $-C_2H_5$ | $n-C_3H_7$ | $-CF_3$ | 0 | 2- | Sdp. 142-146°C/0,53 mbar (0,4 mm Hg) |
| 5 | S | S | $-C_2H_5$ | $n-C_3H_7$ | $-CF_3$ | 0 | 4- | $n_D^{20}$ 1.5357 |
| 6 | O | S | $-C_2H_5$ | $-C_2H_5$ | $-CF_3$ | 0 | 4- | $n_D^{20}$ 1.5021 |
| 7 | O | S | $-C_2H_5$ | $-C_2H_5$ | $-CF_3$ | 2 | 4- | $n_D^{20}$ 1.5075 |
| 8 | S | O | $-C_2H_5$ | $sec-C_4H_9$ | $-CF_3$ | 0 | 4- | $n_D^{20}$ 1.5055 |
| 9 | S | O | $-C_2H_5$ | $-C_2H_4OC_2H_5$ | $-CF_3$ | 0 | 4- | $n_D^{20}$ 1.5045 |
| 10 | NH | S | $-C_2H_5$ | $iso-C_3H_7$ | $-CF_3$ | 0 | 4- | $n_D^{20}$ 1.5106 |
| 11 | O | S | $-C_2H_5$ | $-C_2H_5$ | $-CF_2CHF_2$ | 0 | 4- | $n_D^{20}$ 1.4985 |
| 12 | S | S | $-C_2H_5$ | $n-C_3H_7$ | $-CF_2CHF_2$ | 0 | 4- | $n_D^{20}$ 1.5296 |
| 13 | S | O | $-C_2H_5$ | $sec-C_4H_9$ | $-CF_2CHF_2$ | 0 | 4- | $n_D^{20}$ 1.5030 |

Synthese-Beispiel 4

O,O-Diethyl-O-[4-(2,2,2-trifluoroethylsufonyl)-phenyl] thiophosphat, die in der Tabelle 10 aufgeführte Verbindung Nr. 7 der vorliegenden Erfindung, kann in einfacher Weise auch durch 2-Stündiges Erhitzen von 1 mol O,O-Diethyl-O-[4-(2,2,2-trifluoroethylthio)-phenyl] thiophosphat, der Verbindung Nr. 6 der vorliegenden Erfindung, zusammen mit 2,0 bis 2,2 mol wäßrigem Hydrogenperoxid in Essigsäure auf 30 °C bis 50 °C erhalten werden ; eine weitere Möglichkeit zur Synthese dieser Verbindung ist eine gewöhnliche Oxidations-Reaktion, bei der die Verbindung Nr. 6 mit 2,0 bis 2,2 mol m-Chloroperoxybenzoesäure in Chloroform 2 h zum Rückfluß erhitzt wird. Eine physikalische Konstante des Produkts ist, wie in Tabelle 10 angegeben, $n_D^{20}$ = 1,507 5.

Synthese-Beispiel 5

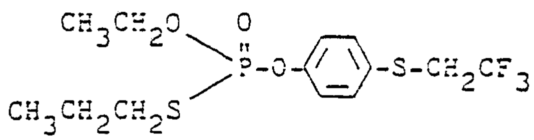

(Verbindung Nr. 3)

Eine Mischung aus 3,7 g Kalium-O-ethyl-O-[4-(2,2,2-trifluoroethylthio) phenyl] thiophosphat, 1,4 g Propylbromid und 30 ml Acetonitril wurde 3 h bei 50 °C bis 60 °C gerührt. Das Acetonitril wurde abgedampft, und Wasser wurde zu dem Rückstand hinzugefügt. Die Mischung wurde gerührt, und die erhaltene ölige Substanz wurde mit 50 ml Toluol extrahiert. Die Toluol-Schicht wurde mit zweimal je 50 ml einer gesättigten wäßrigen Natriumhydrogencarbonat-Lösung und danach mit Wasser gewaschen. Die organische Schicht wurde abgetrennt und über wasserfreiem Natriumsulfat entwässert. Das Toluol wurde abgedampft, und der Rückstand wurde unter vermindertem Druck destilliert, wonach 2,8 g des angestrebten O-Ethyl-S-propyl-O-[4-(2,2,2-trifluoroethylthio)-phenyl] thiophosphats erhalten wurden ; Sdp. 157-160 °C/1,07 mbar (0,8 mm Hg) ; $n_D^{20}$ = 1,505 0.

**Patentansprüche**

1. Organophosphat-Derivat der allgemeinen Formel (I),

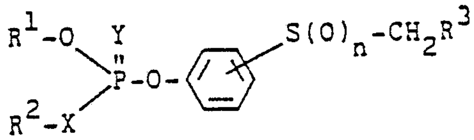

(I)

in der

X für O, S oder NH steht,
Y für O oder S steht,
$R^1$ eine niedere Alkyl-Gruppe bezeichnet,
$R^2$ eine niedere Alkyl-Gruppe oder eine niedere Alkoxy-Niederalkyl-Gruppe bezeichnet,
$R^3$ eine fluoro-substituierte niedere Alkyl-Gruppe bezeichnet und
n für 0 oder 2 steht.

2. Organophosphat-Derivat nach Anspruch 1, dadurch gekennzeichnet, daß
$R^1$ Alkyl mit 1 bis 4 Kohlenstoff-Atomen bezeichnet,
$R^2$ Alkyl mit 1 bis 4 Kohlenstoff-Atomen oder Alkoxyalkyl mit jeweils 1 bis 4 Kohlenstoff-Atomen in der Alkyl- und der Alkoxy-Struktureinheit bezeichnet,
$R^3$ fluorosubstituiertes Alkyl mit 1 bis 4 Kohlenstoff-Atomen ; das durch 1 bis 4 Fluor-Atome substituiert ist, bezeichnet,
X für O, S oder NH steht,
Y für S oder O steht und
n für 0 oder 2 steht.

3. Organophosphat-Derivat nach Anspruch 1, dadurch gekennzeichnet, daß
$R^1$ Methyl oder Ethyl bezeichnet,
$R^2$ Methyl, Ethyl, n- und i-Propyl, sec-Butyl oder Ethoxyethyl bezeichnet,
$R^3$ Trifluoromethyl oder 1,1-Difluoro-2,2-difluoroethyl bezeichnet,
X für O, S oder NH steht,
Y für O oder S steht,
n für 0 oder 2, insbesondere für 0, steht und

die $S(O)_n—CH_2—R^3$-Gruppe sich in der 2- oder 4-Stellung, insbesondere in der 4-Stellung, des Phenoxy-Rings befindet.

4. Organophosphat-Derivat nach Anspruch 1 bis 3, dadurch gekennzeichnet, daß n für 0 steht.

5. Organophosphat-Derivat nach Anspruch 1 bis 3, dadurch gekennzeichnet, daß die $S(O)_n—CH_2—R^3$-Gruppe sich in der 4-Stellung des Phenoxy-Rings befindet.

6. Phenol-Derivat der allgemeinen Formel (III)

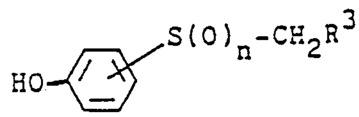

(III)·

in der

$R^3$ eine fluoro-substituierte niedere Alkyl-Gruppe bezeichnet und

n für 0 oder 2 steht.

7. Verfahren zur Herstellung eines Organophosphat-Derivats der allgemeinen Formel (I)

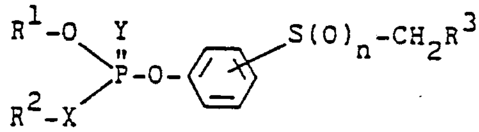

(I)

in der

X für O, S oder NH steht,

Y für O oder S steht,

$R^1$ eine niedere Alkyl-Gruppe bezeichnet,

$R^2$ eine niedere Alkyl-Gruppe oder eine niedere Alkoxy-Niederalkyl-Gruppe bezeichnet,

$R^3$ eine fluoro-substituierte niedere Alkyl-Gruppe bezeichnet und

n für 0 oder 2 steht,

dadurch gekennzeichnet, daß

a) ein Phosphorsäureester-halogenid der allgemeinen Formel (II)

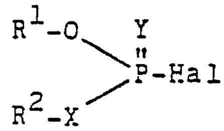

(II)

in der

X, Y, $R^1$ und $R^2$ die im Vorstehenden angegebenen Bedeutungen haben und

Hal ein Halogen-Atom bezeichnet,

mit einem Phenol der allgemeinen Formel (III)

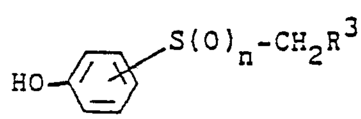

(III)

in der

$R^3$ und n die im Vorstehenden angegebenen Bedeutungen haben,

zur Reaktion gebracht wird, oder

b) (zur Gewinnung von Verbindungen der Formel (I), in der X = S) ein Phosphorsäure-Salz der allgemeinen Formel (IV)

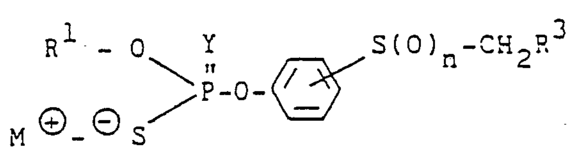

(IV)

in der

Y, $R^1$, $R^3$ und n die im Vorstehenden angegebenen Bedeutungen haben und

M für ein Alkalimetall oder eine Ammonium-Gruppe steht,

mit einem Halogenid der allgemeinen Formel (V)

$R^2$—Hal (V)

in der

$R^2$ die im Vorstehenden angegebene Bedeutung hat und

Hal ein Halogen-Atom bezeichnet,

zur Reaktion gebracht wird, oder

c) ein Phosphorsäureester-halogenid der allgemeinen Formel (VI)

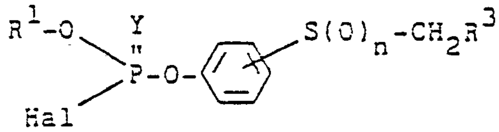

(VI)

in der

Y, $R^1$, $R^3$, n und Hal die im Vorstehenden angegebenen Bedeutungen haben,

mit einer Verbindung der allgemeinen Formel (VII)

$R^2$—X—H (VII)

in der

X und $R^2$ die im Vorstehenden angegebenen Bedeutungen haben,

zur Reaktion gebracht wird, oder

d) (zur Gewinnung von Verbindungen der Formel (I), in der n = 2) ein Organophosphat-Derivat der allgemeinen Formel (I-a)

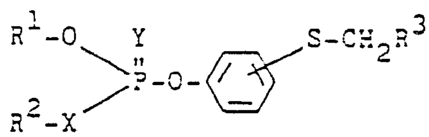

(I-a)

in der

X, Y, $R^1$, $R^2$ und $R^3$ die im Vorstehenden angegebenen Bedeutungen haben,

mit einem Peroxid zur Reaktion gebracht wird.

8. Verfahren zur Herstellung eines Phenol-Derivats der allgemeinen Formel (III)

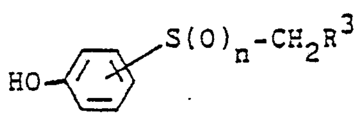

(III)

in der

$R^3$ eine fluoro-substituierte niedere Alkyl-Gruppe bezeichnet und

n für 0 oder 2 steht, dadurch gekennzeichnet, daß

a) (zur Gewinnung von Verbindungen der Formel (III), in der n = 0) ein Sulfonat der allgemeinen Formel (VIII)

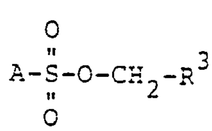

(VIII)

in der

$R^3$ die im Vorstehenden angegebene Bedeutung hat und

A eine Alkyl- oder Aryl-Gruppe bezeichnet,

mit einem Hydroxyphenylthiol der Formel (IX)

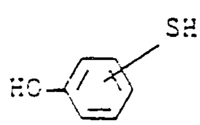

(IX)

zur Reaktion gebracht wird, oder

b) (zur Gewinnung von Verbindungen der Formel (III), in der n = 2) ein Phenol-Derivat der allgemeinen Formel (III-a)

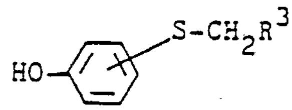

(III-a)

in der

$R^3$ die im Vorstehenden angegebene Bedeutung hat, mit einem Peroxid zur Reaktion gebracht wird.

9. Pestizides Mittel, enthaltend wenigstens ein Organophosphat-Derivat der Formel (I) nach Anspruch 1 bis 5 oder 7.

10. Verwendung von Organophosphat-Derivaten der Formel (I) nach Anspruch 1 bis 5 oder 7 als Schädlingsbekämpfungsmittel.

**Claims**

1. Organophosphate derivative of the general formula (I),

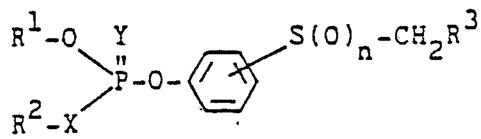

(I)

in which

X represents O, S or NH,

Y represents O or S,

$R^1$ designates a lower alkyl group,

$R^2$ designates a lower alkyl group or a lower alkowy-lower alkyl group,

$R^3$ designates a fluoro-substituted lower alkyl group and

n represents 0 or 2.

2. Organophosphate derivative according to Claim 1, charcterised in that

$R^1$ designates alkyl with 1 to 4 carbon atoms,

$R^2$ designates alkyl with 1 to 4 carbon atoms or alkoxyalkyl with 1 to 4 carbon atoms in the alkyl structural unit and 1 to 4 carbon atoms in the alkoxy structural unit,

$R^3$ designates fluoro-substituted alkyl with 1 to 4 carbon atoms, which is substituted by 1 to 4 fluorine atoms,

X represents O, S or NH,

Y represents S or O and

n represents 0 or 2.

3. Organophosphate derivative according to Claim 1, characterised in that

$R^1$ designates methyl or ethyl,

$R^2$ designates methyl, ethyl, n- or i-propyl, sec-butyl or ethoxyethyl,

$R^3$ designates trifluoromethyl or 1,1-difluoro-2,2-difluoroethyl,

X represents O, S or NH,

Y represents O or S,

n represents 0 or 2, in particular 0, and the $S(O)_n—CH_2—R^3$ group is located in the 2- or 4-position, in particular in the 4-position, of the phenoxy ring.

4. Organophosphate derivative according to Claim 1 to 3, characterised in that n represents 0.

5. Organophosphate derivative according to Claim 1 to 3, characterised in that the $S(O)_n—CH_2—R^3$ group is located in the 4-position of the phenoxy ring.

6. Phenol derivative of the general formula (III)

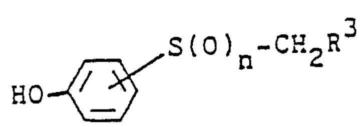

(III)

in which

$R^3$ designates a fluoro-substituted lower alkyl group and

n represents 0 or 2.

7. Process for the preparation of an organophosphate derivative of the general formula (I)

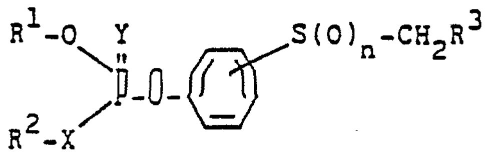

(I)

in which

X repO, S or NH,

Y repO or S,

$R^1$ de a lower alkyl group,

$R^2$ de a lower alkyl group or a lower alkoxy-lower alkyl group,

$R^3$ de a fluoro-substituted lower alkyl group and

n rep0 or 2,

characterhat

a) a ric acid ester halide of the general formula (II)

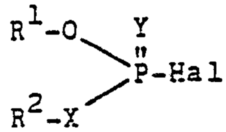

(II)

in which

X, Y,$R^2$ have the meanings indicated above and Hal designates a halogen atom,

is reactephenol of the general formula (III)

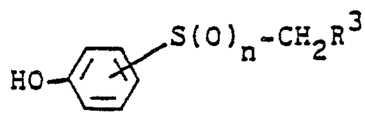

(III)

in which

$R^3$ are the meanings indicated above,

or

b) (tcompounds of the formula (I) in which X = S) a phosphoric acid salt of the general formula (

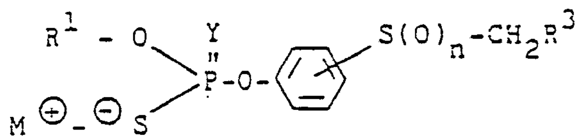

(IV)

in which

Y, $R^1$n have the meanings indicated above and M represents an alkali metal or an ammonium group,

is reactehalide of the general formula (V)

$$R^2\text{—Hal} \qquad (V)$$

in which

$R^2$ heaning indicated above and

Hal is a halogen atom,

or

c) a ric acid ester halide of the general formula (VI)

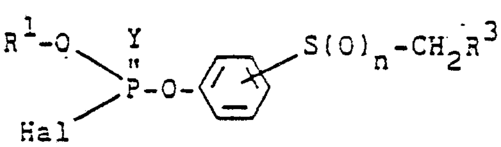

(VI)

in which

Y, $R^1$d Hal have the meanings indicated above, is reacted with a compound of the general formula (

$R^2—X—H$ (VII)

in which

X and $R^2$ have the meanings indicated above,

or

d) (to obtain compounds of the formula (I) in which n = 2) an organophosphate derivative of the general formula (I-a)

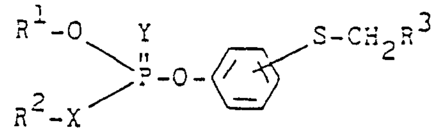

(I-a)

in which

X, Y, $R^1$, $R^2$ and $R^3$ have the meanings indicated above,

is reacted with a peroxide.

8. Process for the preparation of a phenol derivative of the general formula (III)

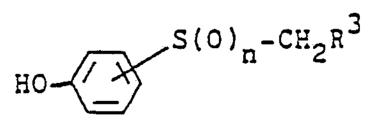

(III)

in which

$R^3$ designates a fluoro-substituted lower alkyl group and

n represents 0 or 2,

characterised in that

a) (to obtain compounds of the formula (III) in which n = 0) a sulphonate of the general formula (VIII)

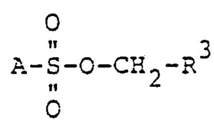

(VIII)

in which

$R^3$ has the meaning indicated above and

A designates an alkyl or aryl group,

is reacted with a hydroxyphenylthiol of the formula (IX)

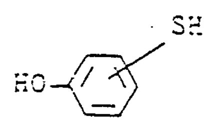

(IX)

or

b) (to obtain compounds of the formula (III) in which n = 2) a phenol derivative of the general formula

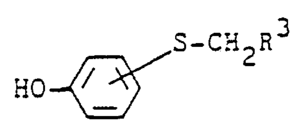

(III-a)

in which

$R^3$ has the meaning indicated above,

is reacted with a peroxide.

9. Pesticidal agent, containing at least one organophosphate derivate of the formula (I) according to Claim 1 to 5 or 7.

10. Use of organosphosphate derivatives of the formula (I) according to Claim 1 to 5 or 7 as pest-combating agents.

**Revendications**

1. Dérivé du type d'un phosphate organique de formule générale (I)

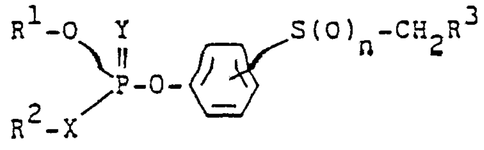

(I)

dans laquelle

X représente O, S ou NH,

Y représente O ou S,

$R^1$ désigne un groupe alkyle inférieur,

$R^2$ désigne un groupe alkyle inférieur ou un groupe (alkoxy inférieur)-alkyle inférieur,

$R^3$ désigne un groupe alkyle inférieur à substituant fluoro et

n a la valeur 0 ou 2.

2. Dérivé du type d'un phosphate organique suivant la revendication 1, caractérisé en ce que

$R^1$ est un groupe alkyle ayant 1 à 4 atomes de carbone,

$R^2$ est un groupe alkyle ayant 1 à 4 atomes de carbone ou un groupe alkoxyalkyle ayant 1 à 4 atomes de carbone dans le motif structural alkyle ainsi que dans le motif structural alkoxy,

$R^3$ est un groupe alkyle à substitution fluoro ayant 1 à 4 atomes de carbone, qui est substitué par 1 à 4 atomes de fluor,

X représente O, S ou NH,

Y représente S ou O et

n a la valeur 0 ou 2.

3. Dérivé du type phosphate organique suivant la revendication 1, caractérisé en ce que

$R^1$ désigne le groupe méthyle ou éthyle,

$R^2$ désigne le groupe méthyle, éthyle, n-propyle, isopropyle, sec.-butyle ou éthoxyéthyle,

$R^3$ désigne le groupe trifluorométhyle ou 1,1-difluoro-2,2-difluoréthyle,

X représente O, S ou NH,

Y représente O ou S,

n a la valeur 0 ou 2, notamment la valeur 0, et le groupe $S(O)_n—CH_2—R^3$ se trouve en position 2 ou 4, notamment en position 4 du noyau phénoxy.

4. Dérivé du type phosphate organique suivant les revendications 1 à 3, caractérisé en ce que n est égal à 0.

5. Dérivé du type phosphate organique suivant les revendications 1 à 3, caractérisé en ce que le groupe $S(O)_n—CH_2—R^3$ se trouve en position 4 du noyau phénoxy.

6. Dérivé phénolique de formule générale (III)

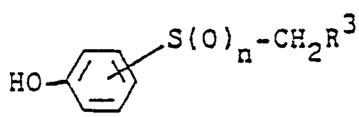

(III)

dans laquelle

$R^3$ est un groupe alkyle inférieur à substitution fluoro et

n a la valeur 0 ou 2.

7. Procédé de production d'un dérivé du type d'un phosphate organique de formule générale (I)

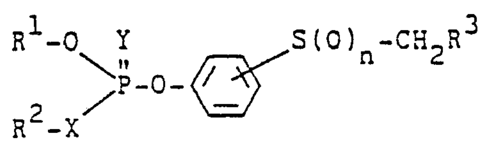

(I)

dans laquelle

X représente O, S ou NH,

Y représente O ou S,

$R^1$ désigne un groupe alkyle inférieur,

$R^2$ désigne un groupe alkyle inférieur ou un groupe (alkoxy inférieur)-alkyle inférieur,

$R^3$ désigne un groupe alkyle inférieur à substituant fluoro et

n a la valeur 0 ou 2,

caractérisé en ce que

a) on fait réagir un halogénure d'ester d'acide phosphorique de formule générale (II)

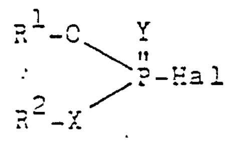

(II)

dans laquelle
X, Y, $R^1$ et $R^2$ ont les définitions indiquées ci-dessus et Hal désigne un atome d'halogène, avec un phénol de formule générale (III)

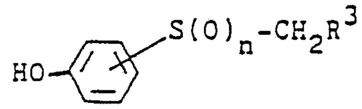

(III)

dans laquelle
$R^3$ et n ont les définitions indiquées ci-dessus,
ou bien
b) (pour l'obtention de composés de formule (I), dans laquelle X représente S), on fait réagir un sel d'acide phosphorique de formule générale (IV)

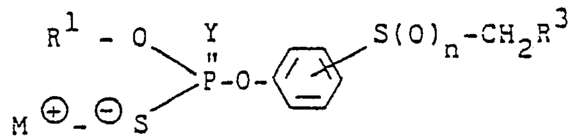

(IV)

dans laquelle
Y, $R^1$, $R^3$ et n ont les définitions indiquées ci-dessus et
M représente un métal alcalin ou un groupe ammonium, avec un halogénure de formule générale (V)

$$R^2\text{—Hal} \qquad (V)$$

dans laquelle
$R^2$ a la définition indiquée ci-dessus, et
Hal désigne un atome d'halogène,
ou bien
c) on fait réagir un halogénure d'ester d'acide phosphorique de formule générale (VI)

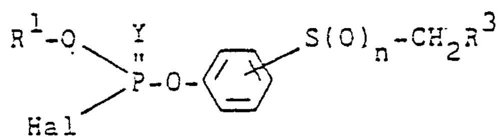

(VI)

dans laquelle
Y, $R^1$, $R^3$, n et Hal ont les définitions indiquées ci-dessus, avec un composé de formule générale (VII)

$$R^2\text{—X—H} \qquad (VII)$$

dans laquelle
X et $R^2$ ont les définitions indiquées ci-dessus,
ou bien
d) (pour l'obtention de composés de formule (I), dans laquelle n est égal à 2), on fait réagir avec un peroxyde un dérivé organophosphorique de formule générale (I-a)

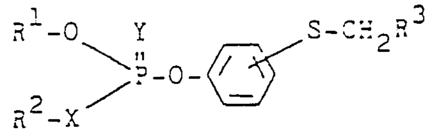

(I-a)

dans laquelle
X, Y, $R^1$, $R^2$ et $R^3$ ont les définitions indiquées ci-dessus.

8. Procédé de production d'un dérivé phénolique de formule générale (III)

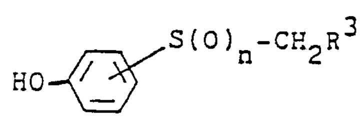

(III)

dans laquelle

$R^3$ est un groupe alkyle inférieur à substitution fluoro et
n a la valeur 0 ou 2,
caractérisé en ce que
a) (pour l'obtention de composés de formule (III), dans laquelle n est égal à 0), on fait réagir un sulfonate de formule générale (VIII)

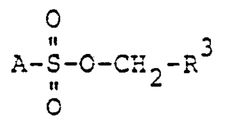

(VIII)

dans laquelle
$R^3$ a la définition indiquée ci-dessus et
A désigne un groupe alkyle ou aryle,
avec un hydroxyphénylthiol de formule (IX)

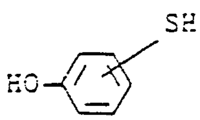

(IX)

ou bien
b) (pour l'obtention de composés de formule (III), dans laquelle n est égal à 2), on fait réagir avec un peroxyde un dérivé phénolique de formule générale (III-a)

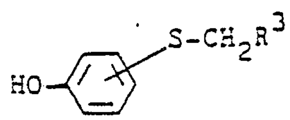

(III-a)

dans laquelle
$R^3$ a la définition indiquée ci-dessus.

9. Composition pesticide, contenant au moins un dérivé du type d'un phosphate organique de formule (I) suivant les revendications 1 à 5 ou 7.

10. Utilisation de dérivés du type de phosphates organiques de formule (I) suivant les revendications 1 à 5 ou 7, comme pesticides.